# EUROPEAN PATENT APPLICATION

(11) **EP 2 508 544 A1**
(43) Date of publication of application: **10.10.2012**
(21) Application number: 11382097.1
(22) Date of filing: 04.04.2011
(51) Int. Cl.: C08G 2/30, C08G 4/00, C08L 59/00, A61K 47/00, C08F 297/02

(54) **Degradable polyacetal polymers**

(71) Applicant: Argon Pharma S.L., 08028 Barcelona (ES)
(72) Inventor: Pena Gulin Oscar, 08027, BARCELONA (ES)
(74) Representative: ZBM Patents

(57) **Abstract**

The present invention relates to degradable polyacetal polymers having a C(O)-(CH₂CH₂O)ₙ-R₅ or a saccharide attached to amine radical of the backbone of the polymer, their polymer-active agent conjugates and their preparation processes, to compositions, and hydrogels containing them, as well as their use in therapy, diagnostic imaging and cosmetics.

## Description

The present invention relates to degradable polyacetal polymers having a C(O)-(CH₂CH₂O)ₙ-R₅ or a saccharide attached to amine radical of the backbone of the polymer, their polymer-active agent conjugates and their preparation processes, to compositions, and hydrogels containing them, as well as their use in therapy, diagnostic and cosmetics.

### BACKGROUND ART

Therapeutic polymers have been widely developed for biomedical applications. These polymers require a suitable solubility in the physiological media and an appropriate degradation profile for being stable during their targeting and allowing the release of the active agent in the target sites. These therapeutic polymers can include biologically active polymers, polymer-drug conjugates, polymer-protein conjugates, and other covalent constructs of bioactive molecules.

These therapeutic polymers should accumulate only in the target sites. In particular, they are accumulated in tumors by active or passive targeting. The higher vascularization and the lack of an efficient lymphatic drainage in tumor tissues is known as Enhanced Permeability and Retention effect (EPR effect), and enables the passive accumulation in such tissues. Thus, the molecular weight of the polymer conjugate in blood, after its administration, should be higher than the renal threshold and similar to large protein size.

A high number of degradable polymers useful as macromolecular partners for the conjugation of bioactive molecules have been developed. Many of these polymers are soluble but they have the disadvantage of being non-degradable in the physiological conditions. The degradable polymers should be stable in the bloodstream and also in the extracellular conditions allowing their accumulation, while they should be rapidly degraded once the polymer is internalized into the target cell.

There are known hydrophilic soluble copolymers containing monomers of poly(ethylene glycol) and hydroxypropyl methacrylamide (HPMA) in the backbone which does not degrade in vivo.

Other synthetic polymers which contains potentially degradable monomers derived from amino acids (i.e. poly(glutamic acid), poly[⁵N-(2-hydroxyethyl)-L-glutamine)], beta-poly(2-hydroxyaspartamide), and polylysine) have been prepared. Nevertheless, the corresponding polymer conjugates based on the above polymers, whose degradation dependents on enzymatic activity, did not degrade in vivo to any extent within a time period of from 10 to 100 hours.

Acetals are well known to be hydrolytically labile under mildly acidic conditions. Thus, biomedical polymers containing acetal linkages in the polymer backbone may undergo enhanced rates of hydrolysis in biological environments that are mildly acidic compared to biological environments that are at neutral or basic pH. Soluble polyacetals that can conjugate a bioactive molecule are expected to degrade at enhanced rates at the acetal functionality during cellular uptake because of the increase in acidity during endocytosis. Polyacetals will also display enhanced rates of hydrolysis in acidic regions of the gastrointestinal tract. Additionally polyacetals would be expected to degrade at enhanced rates at sites of diseased tissue that are mildly acidic such as solid tumors.

Polyacetal polymer can be prepared by the reaction of diols and divinyl ether monomers using a catalyst without generation of a small molecule byproduct. These polymers thus obtained have a uniform structure in which there is a strict alternation of such monomers (A-B type).

European patent number EP1315777 discloses degradable polyacetal polymers containing monomers X and monomers Y. Monomer X is a group capable of being covalently conjugated to a bioactive agent via a peptidic or a hydrolytically-labile bond, and monomers Y is a hydrocarbon group. In particular, polyacetal 22 has been exemplified having a molecular weight of 23,000 g/mol and contains repeating units of poly(ethylene glycol) monomers of formula:

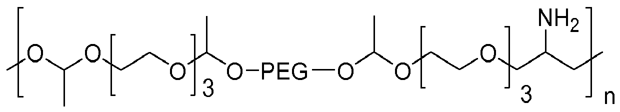

Polyacetal 22 is degraded more rapidly in the mildly acidic medium at pH 5.5 than in the relatively neutral physiological at pH 7.4. The degradation profile of this amino polyacetal was determined by the loss in the molecular weight according to the time. Nevertheless, it is silent of its percentage of accumulation around the target cells, and its degradation profile at extracellular conditions (pH 6.5).

Therefore, from what is known in the art it is derived that there is still the need of providing degradable polymers having an improved control of the degradation of the polymer for biomedical applications.

### SUMMARY OF THE INVENTION

Inventors have found that a degradable polymers having a molecular weight of from 1,000 to 1,000,000 g/mol which comprises a backbone of repeating structural units, being these repeating structural units of formula (I) equals or differents, wherein the amine groups of the lateral chains of the repeating units of the backbone can be substituted by C(O)-(CH₂CH₂O)ₙ-R₅ or a saccharide having at least a monosaccharide with a carboxylic group, selected from monosaccharide, disaccharide, and oligosaccharide are in a percentage of from 1 to 50% by weight of the weight of the polymer, are useful for an improved control of pH-dependent degradation of the polymer allowing the controlled release of the active agents bound to the polymer in the target site.

The structure of the degradable polymer of the present invention is also advantageous for their versatility and multifunctionality which allows the loading of a combination of several active agents from small agents (e.g. pharmaceutical active ingredients, antioxidants or diagnostic imaging agents) to big size molecules (e.g. peptides, enzymes or antibodies). These active agents can be covalently or non-covalently bound to the polymer. It allows the development of more effective combined therapy when a multi-drug system is needed, for example in the treatment of cancer.

Thus, a first aspect of the present invention relates to a polymer having a molecular weight of from 1,000 to 1,000,000 g/mol which comprises a backbone of repeating structural units, being these repeating structural units equals or differents of formula (I), 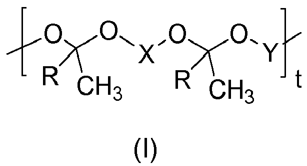 where: R is a radical independently selected from the group consisting of hydrogen, and C₁-C₄ alkyl; X is a birradical selected from the group consisting of formula (II), (III), (IV), (V), (VI), (VII), (VIII), and (IX); 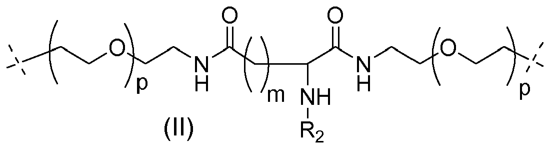 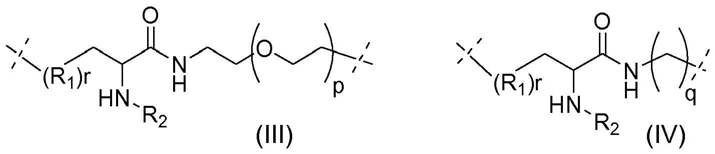 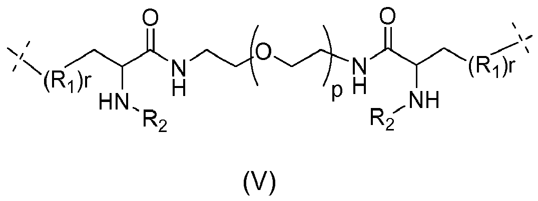 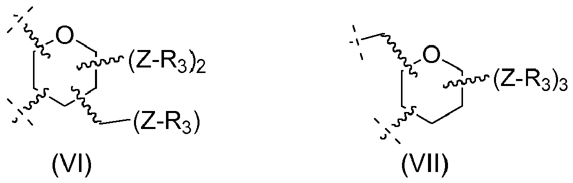 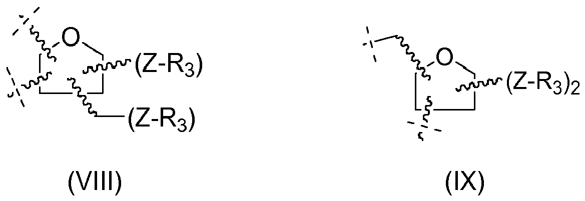 Y is a birradical selected from the group consisting of formula (X), and (XI); 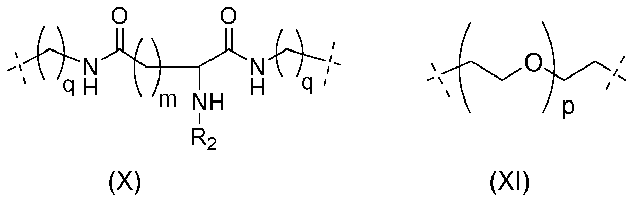 Z is a heteroatom selected from the group consisting of N and O; R₁ is a phenyl group; R₂ is selected from the group consisting of hydrogen, C₁-C₄ alkyl, C(O)-(CH₂)ᵤ-OH, an amine protecting group, C(O)-(CH₂CH₂O)ₙ-R₅, and a saccharide, having at least a monosaccharide with a carboxylic group, selected from the group consisting of monosaccharide, disaccharide, and oligosaccharide; R₃ is selected from the group consisting of of hydrogen, C₁-C₄ alkyl, C(O)-(CH₂)ᵤ-OH, an amine or hydroxy protecting group, C(O)-(CH₂CH₂O)ₙ-R₅, and a saccharide, having at least a monosaccharide with a carboxylic group, selected from the group consisting of monosaccharide, disaccharide, and oligosaccharide, with the proviso that when Z is O then R₃ is a hydroxyl protecting group, hydrogen or C₁-C₄ alkyl; R₅ is selected from the group consisting of hydrogen, and C₁-C₄ alkyl; m is an integer from 1 to 2; n is an integer from 1 to 300; p is an integer from 1 to 20; q is an integer from 1 to 20; r is an integer from 0 to 1; t is an integer from 1 to 2,000; u is an integer from 1 to 20; the wavy line indicates any of the two possible configurations of the radicals attached to the carbocycle of formula (VI), (VII), (VIII), and (IX); the intermittent line indicates the position through which the birradicals X and Y bind to the oxygen atoms of the repeating structural units of the backbone, with the proviso that R₂ or R₃ is C(O)-(CH₂CH₂O)ₙ-R₅ or the saccharide in a percentage of from 1 to 50% by weight of the weight of the polymer.

A second aspect of the present invention relates to a polymer-active agent conjugate which comprises the polymer as defined above and at least an active agent being covalently bound directly or by one or two linkers to the backbone, where: a) the active agent is a radical attached to the amine radical of the backbone when R₂ is hydrogen or C₁-C₄ alkyl, or when Z-R₃ is NH or N(C₁-C₄ alkyl), or alternatively, b) the active agent is a birradical which replaces the birradical X or the birradical Y; or alternatively, c) a mixture of a) and b).

A third aspect of the present invention relates to a process for preparing a polymer as defined above, which comprises: a) reacting a diol selected from the group consisting of formula (XII), (XIII), (XIV), (XV), (XVI), (XVII), (XVIII), and (XIX) 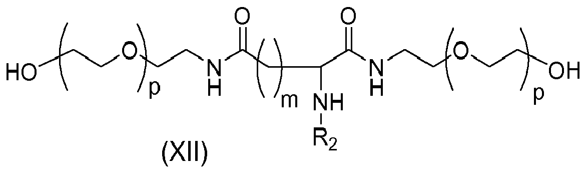 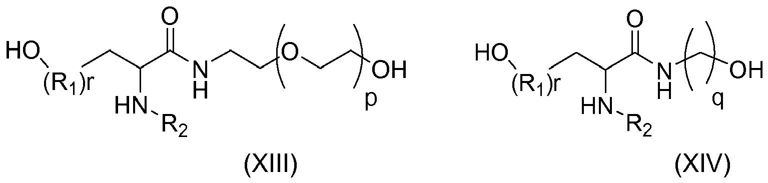 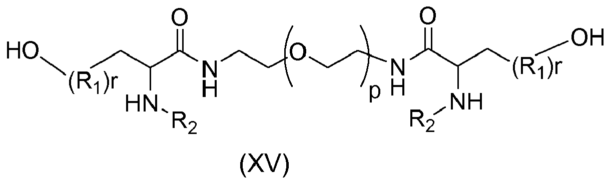 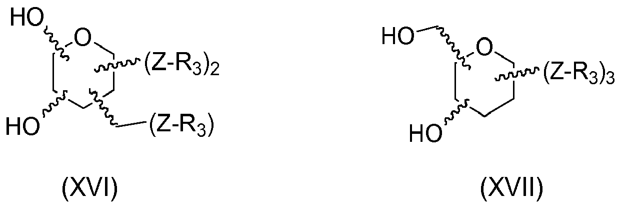 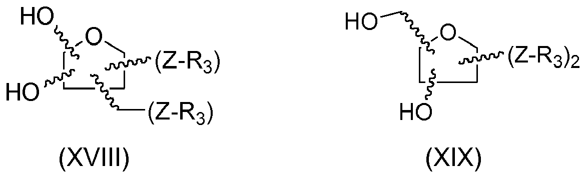 with a divinyl ether selected from the compound of formula (XX), and (XXI), 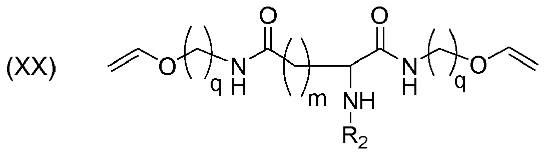 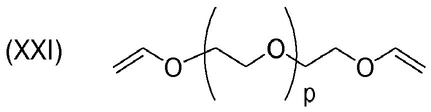 in an anhydrous organic solvent in the presence of an acid; b) deprotecting the amine groups of the polymer-active agent conjugate obtained in step (a); and c) reacting the polymer obtained in step (b) with the compound of formula (XXII), 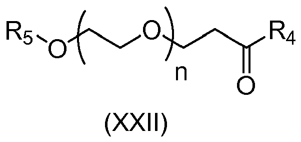 or alternatively with a saccharide, having at least a monosaccharide with a carboxylic group, selected from the group consisting of monosaccharide, disaccharide, and oligosaccharide; where: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; R₅ is selected from the group consisting of hydrogen, and C₁-C₄ alkyl; and m, n, p, q, r, u, Z, and R₁ are as defined above.

A fourth aspect of the present invention relates to a process for preparing a polymer-active agent conjugate as defined above, which comprises: a) reacting the diol selected from the group consisting of formula (XII), (XIII), (XIV), (XV), (XVI), (XVII), (XVIII), and (XIX) ; with the divinyl ether selected from the group consisting of formula (XX), and (XXI); and the active agent in an anhydrous organic solvent in the presence of an acid; b) deprotecting the amine groups of the polymer-active agent conjugate obtained in step (a); and c) reacting the polymer-active agent conjugate obtained in step (b) with the compound selected from the group consisting of (XXII), and a saccharide having a carboxylic group; or alternatively, a') reacting the diol selected from the group consisting of formula (XII), (XIII), (XIV), (XV), (XVI), (XVII), (XVIII), and (XIX) with the divinyl ether selected from the group consisting of formula (XX), and (XXI), in an anhydrous organic solvent in the presence of an acid; b') deprotecting the amine groups of the polymer obtained in step (a'); c') reacting the polymer obtained in step (b') with the active agent; and d') reacting the polymer-active agent conjugate obtained in step (c') with the compound selected from the group consisting of (XXII), and a saccharide having a carboxylic group; or alternatively, a") reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid; b") deprotecting the amine groups of the polymer-active agent conjugate obtained in step (a"); c") reacting the polymer obtained in step (b") with the active agent; and d") reacting the polymer-active agent conjugate obtained in step (c") with the compound selected from the group consisting of (XXII), and a saccharide having a carboxylic group; or alternatively, wherein: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; and m, n, p, q, r, u, Z, R₁, and R₅ are as defined above.

A fifth aspect of the present invention relates to a pharmaceutical or diagnostic imaging or cosmetic composition which comprises the polymer-active agent conjugate defined above, together with one or more appropriate pharmaceutically, diagnostically imaging or cosmetically acceptable excipients or carriers respectively.

A sixth aspect of the present invention relates to a polymer-active agent conjugate defined above, where the active agent is a pharmaceutical active ingredient for use in therapy.

A seventh aspect of the present invention relates to a polymer-active agent conjugate defined above, where the active agent is a contrast agent for use as diagnostic imaging agent.

A eighth aspect of the present invention relates to the use of a polymer-active agent conjugate as defined above, wherein the active agent is a cosmetic agent as a skin care agent, wherein the skin care comprises ameliorating at least one of the following symptoms: roughness, flakiness, dehydration, tightness, chapping, and lack of elasticity.

A ninth aspect of the present invention relates to a hydrogel comprising polymers cross-linked with a cross-linker of formula -CO-CH₂-CH₂-O-(CH₂CH₂O)ₙ-CH₂CH₂CO-, obtainable by the process which comprises cross-linking at least two polymers selected from the group consisting of: a) at least two polymers obtainable by the process defined in step (b) of third aspect; b) at least two polymers obtainable by the process defined in step (c) of third aspect, wherein the polymer obtained in step (b) is reacted with the saccharide as defined in the third aspect; c) at least two polymer-active agent conjugate obtainable by the process defined in step (b) of the fourth aspect; d) at least two polymer-active agent conjugate obtainable by the process defined in step (c) of the fourth aspect; wherein the polymer obtained in step (b) is reacted with the saccharide as defined in the third aspect; e) at least two polymer-active agent conjugate obtainable by the process defined in step (c') of the fourth aspect; f) at least two polymer-active agent conjugate obtainable by the process defined in step (d') of the fourth aspect; wherein the polymer obtained in step (c') is reacted with the saccharide as defined in the third aspect; g) at least two polymer-active agent conjugate obtainable by the process defined in step (c") of the fourth aspect; h) at least two polymer-active agent conjugate obtainable by the process defined in step (d") of the fourth aspect; wherein the polymer obtained in step (c") is reacted with the saccharide as defined in the third aspect; i) a mixture of at least two different polymers selected from (a), (b), (c), (d), (e), (f), (g), and (h); with a compound of formula (XXIII), 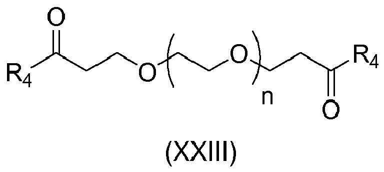 where R₄ is as defined above; and the percentage of the cross-linker in the hydrogel is of from 0.1 to 75% by weight of the weight of the hydrogel.

A tenth aspect of the present invention relates to the hydrogel of the present invention, further comprising an active agent being not covalently bound to the backbone of the polymer, selected from the group consisting of a peptide, a protein, an antibody, and a gene construct for gene therapy.

A eleventh aspect of the present invention relates to a pharmaceutical, diagnostic or cosmetic composition which comprises the hydrogel as defined above, together with one or more appropriate pharmaceutically, diagnostically or cosmetically acceptable excipients or carriers.

A twelfth aspect of the present invention relates to the hydrogel of the present invention which comprises a polymer-active agent as defined above or an active agent being not covalently bound to the backbone of the polymer as defined above, wherein at least one active agent is a pharmaceutical active ingredient for use in therapy.

A thirteenth aspect of the present invention relates to the hydrogel of the present invention for use as a support for detection assays.

Finally, a fourteenth aspect of the present invention relates to the use of the hydrogel of the present invention as a skin care agent, wherein the skin care comprises ameliorating at least one of the following symptoms: roughness, flakiness, dehydration, tightness, chapping, and lack of elasticity, wherein when the hydrogel comprises an active agent, then the active agent is a cosmetic agent.

### BRIEF DESCRIPTION OF THE DRAWINGS

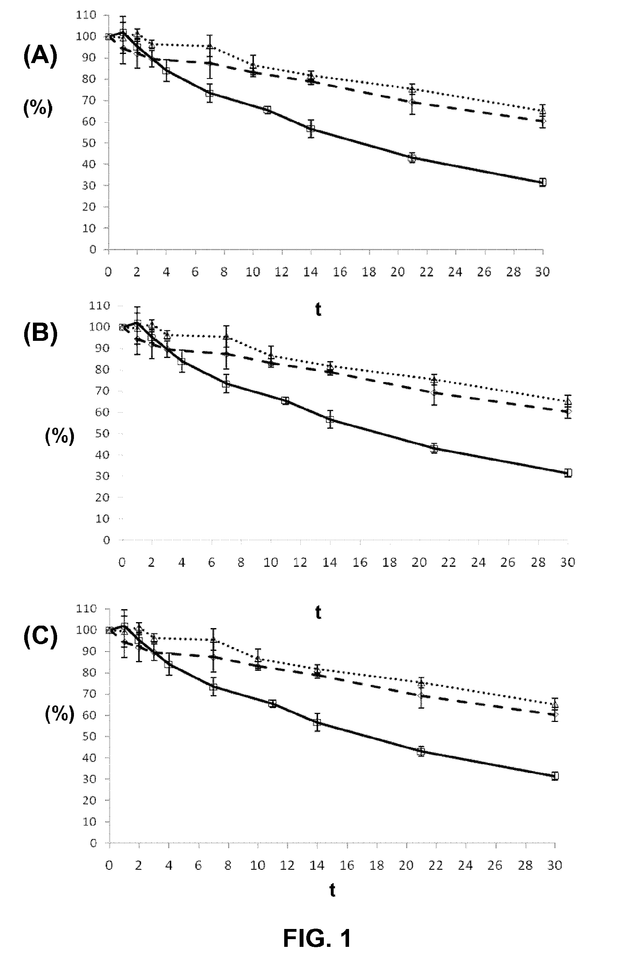

FIG. 1 shows a comparative scheme of the degradation rate of the compounds A, B, and C in an aqueous buffered media at 37°C, and at pH 7.4, pH 6.5, and pH 5.5. The tested compounds are
   - Compound A (PA): polyacetal polymer shown as dotted line (Example 15);
   - Compound B (TPA(DES)): polymer in which DES is inserted in the backbone shown as dashed line (Example 7 section A); and
   - Compound C ([(TPA(DES)]gPEG_{5kDa}): polymer in which DES is inserted in the backbone and PEG attached to the amine radicals of the backbone shown as solid line (Example 7 section B).
Panels of the scheme represent:
Panel (A) shows the percentage of the loss of the molecular weight of the polymer at pH 7.4;
Panel (B) shows the percentage of the loss of the molecular weight of the polymer at pH 6.5; and,
Panel (C) shows the percentage of the loss of the molecular weight of the polymer at pH 5.5.
   The scheme units are shown below: % is the percentage of the loss of the molecular weight of the polymer shown as the relation between Mwₜ/Mwₒ, where Mwₜ is the molecular weight of the polymer at due time, and Mwₒ is the molecular weight of the polymer at time zero; and t is time in days.

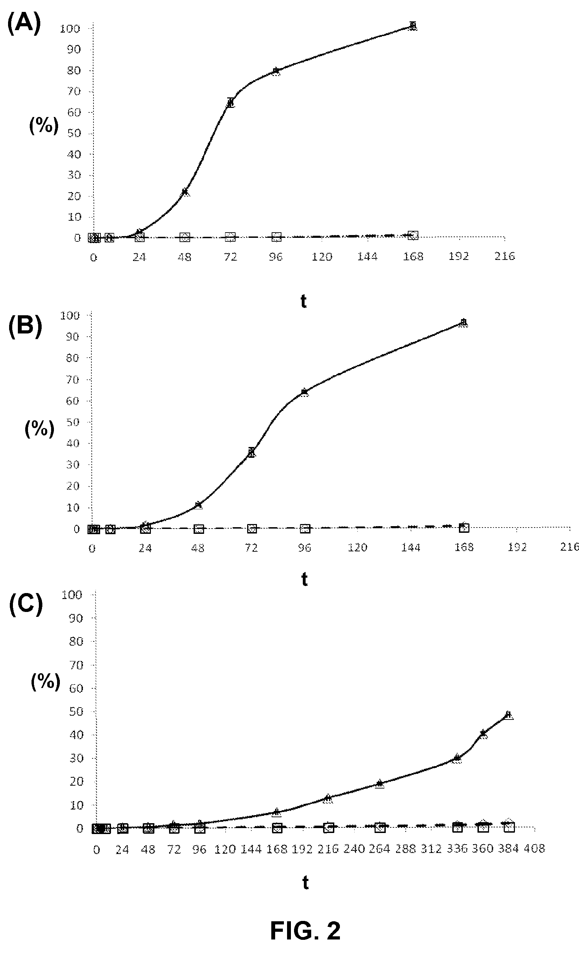

FIG. 2 shows a comparative scheme of the release of floxuridine from compounds D, E, and F in an aqueous buffered media at 37°C, and at the following pH:
   - pH 7.4 shown as dotted line;
   - pH 6.5 shown as dashed line; and
   - pH 5.5 shown as solid line.
   Panels of the scheme represent:
   - Panel (A) shows the percentage of floxuridine released from compound D (TPA(FdUrd)) in which floxuridine is inserted in the backbone of the polymer (Example 8);
   - Panel (B) shows the percentage of floxuridine released from compound E ([TPA(FdUrd)]gPEG_{5kDa}) in which floxuridine is inserted in the backbone and PEG chains are attached to the amine radicals of the backbone (Example 9); and
   - Panel (C) shows the percentage of floxuridine released from compound F (TPA(FdUrd)-NANA) in which floxuridine is inserted in the backbone and N-acetylneuraminic acid chains are attached to the amine radicals of the backbone (Example 10).
   The scheme units are shown below: % is the percentage of floxuridine released from the polymer, and t is time in hours.

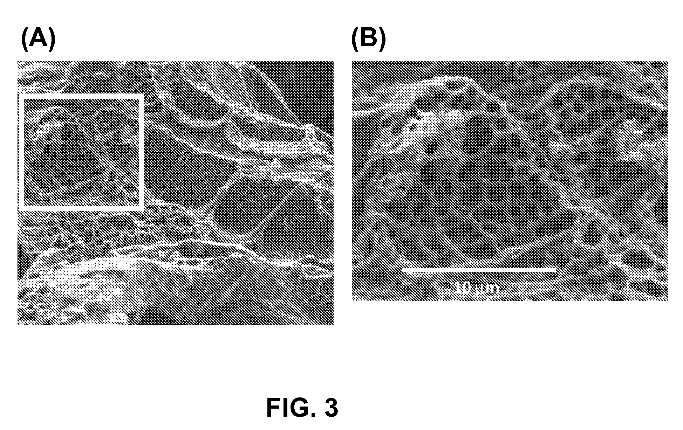

FIG. 3 shows in panel (A) a micrograph image obtained by scanning electron micrograph (SEM) of the hydrogel of the example 14. And, panel (B) is an enlargement of the square marked in the image of panel (A).

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

In the context of the invention, the term C₁-C₄ alkyl refers to a saturated branched or linear alkyl chain which contains from 1 to 4 carbon atoms. Examples include the group methyl, ethyl, propyl, isopropyl, butyl, isobutyl, sec-butyl, and tert-butyl.

The term "saccharide" or "carbohydrate" or "sugar" have the same meaning and are interchangeable. These terms refer to an organic compound which consists only of carbon, hydrogen, and oxygen, with hydrogen: oxygen atom ratio of 2:1. Saccharides are divided into four chemical groups: monosaccharides, disaccharides, oligosaccharides, and polysaccharides. There are derivates of saccharides which contains at least a carboxyl group known as sugar acids and some of them contains also at least an amino moiety

The term "monosaccharide" refers to the most basic units of biologically important carbohydrates. They are the simplest form of sugar, and are usually colorless, water-soluble, and crystalline solids. Some monosaccharides have a sweet taste. Examples of monosaccharide include glucose (dextrose), fructose (levulose), galactose, xylose and ribose. Examples of monosaccharide sugar acids having a carboxylic group include glucuronic acid, galacturonic acid, muramic acid, and neuraminic acid.

The term "disaccharide" refers to two units of monosaccharide obtained by a dehydration reaction that leads to the loss of a molecule of water, and formation of a glycosidic bond. Disaccharides are formed when two monosaccharides are joined together and a molecule of water is removed. Examples of disaccharides include sucrose, lactulose, lactose, maltose, trehalose, and cellobiose.

The term "oligosaccharide" refers to a saccharide polymer containing a small number of monosaccharide; the number of monosaccharides is typically of from three to ten. A suitable oligosaccharide for the preparation of the polymers of the present invention is a cyclodextrin.

The term "antibody" refers to a whole antibody, including without limitation a chimeric, humanized, recombinant, transgenic, grafted and single string, and the like, or any fusion protein, conjugate, fragment, or derivatives thereof containing one or more domains that bind selectively to specific surface proteins. The term antibody also includes a whole immunoglobulin molecule, a monoclonal antibody, or an immunologically effective fragment of any of them. For an antibody fragment means a Fv, a disulfide linked Fv fragments, scFv, Fab, F(ab') or F(ab')2, which are well known in the art. A fragment of an antibody represents any part of it with an appropriate size and conformation to bind to surface proteins. The term "Fv" refers to a fragment of an antibody variable. The term "scFv" refers to a single chain variable fragment corresponding to half of a Fab is present only where the party provides the specificity. The scFv is obtained by joining the variable heavy and light chains of an antibody. The term "Fab" refers to a fragment of an antibody and an antigen binding. By the term "F(ab')" refers to the antibody fragment obtained by digestion of whole antibody with the enzyme pepsin and the term "F(ab')2" refers to the antibody fragment obtained by digestion of a whole antibody with the enzyme papain.

The term "pharmaceutically acceptable" refers to that excipients or carriers suitable for use in the pharmaceutical technology for preparing compositions with medical use.

The term "diagnostically imaging acceptable" refers to that excipients or carriers suitable for use in the imaging diagnosing technology for preparing compositions with imaging diagnostic use.

The term "cosmetically acceptable" or "dermatological acceptable" which is herein used interchangeably refers to that excipients or carriers suitable for use in contact with human skin without undue toxicity, incompatibility, instability, allergic response, among others.

The term "contrast agent" refers to agents that illuminate certain structures that would otherwise be hard to see on the radiograph. These agents are used for injection into the vascular system for either a local visualization of a system or organ or for outlining an excretory system.

The term "cross-linking" refers in the synthetic polymer science field, the use of cross-links to promote a difference in the physical properties of the polymers. The term "cross-links" refers to bonds that link one polymer chain to another and these bonds can be covalent or ionic bonds. The term "cross-linker" or "cross-linking agent" which is herein used interchangeably refers to compound having the ability to cross-link the polymer chains.

The term "cell" refers to a culture of autogenic or allogenic cells being differentiated cells or stem cells. The term "autogenic cells" refers to those cells which come from the same subject, and the term "allogenic cells" refers to those cells originating in other subject.

The term "differentiated cells" refers to those cells which are present in a multicellular organism that is specialized in its functions, which it may perform in a certain organ or tissue. The "stem cells" can be classified according to their differentiation potential as: totipotent, pluripotent and multipotent. Totipotent stem cells can grow and form a complete organism, forming both embryonic components (such as the three germ layers, the germline and the tissues which will give rise to the yolk sac, for example) and extraembryonic components (such as the placenta). In other words, they can form all the cell types. Multipotent stem cells are those which can only generate cells of their own germ layer or germline of origin, for example: since a bone marrow mesenchymal stem cell has a mesodermal nature, it will give rise to cells of that layer such as myocytes, adipocytes or osteocytes, among others. Pluripotent stem cells cannot form a complete organism, but they can differentiate into cells from the three germ layers: (a) ectoderm, which is the origin of the nervous system, the respiratory system, upper digestive tract (stomodeum), the epidermis and its adnexa (hair and nails) and the mammary glands; (b) endoderm, which is the origin of the intestine, the liver, the pancreas, the lungs and most of the internal organs; and (c) mesoderm, which is the origin of the skeletal system, the muscles and the circulatory and reproductive systems. They can also form any other type of cell from the germ and the yolk sac.

As mentioned above, an aspect of the present invention refers to a degradable polymer having a molecular weight of from 1,000 to 1,000,000 g/mol which comprises a backbone of repeating structural units, being this repeating structural units equals or differents of formula (I), with the proviso that R₂ or R₃ is C(O)-(CH₂CH₂O)ₙ-R₅ or the saccharide in a percentage of from 1 to 50% by weight of the weight of the polymer.

In comparison with the degradable polyacetal polymers known in the art, the polyacetal polymers of the present invention containing the repeating units of formula (I) are stable in the environmental conditions of the bloodstream, that is pH 7.4, as well as in the extracellular conditions at pH 6.5.

As it is shown in Example 16 Table 1, and in Fig. 1 panel (A), polymers of the invention are more stable than polymers which do not contain free amine radicals in their structure at pH 7.4. In particular, the reduction of the loss of the molecular weight of the polymers of the present invention is about a 35% in relation to Compound A. Additionally, as it is also shown in Example 16 Table 2, and Fig. 1 panel (B) polymers of the invention are more stable in the extracellular media than polymers which does not contain PEG chains (cf. compound B, example 7 section A), and much more stable than polyacetal polymers without free amine radicals and PEG chains.

It means that polymers of the present invention are more stable in the bloodstream and in the extracellular media than the polymers of the state of the art. This is advantageous because it involves that the polymers of the present invention can be accumulated in the extracellular media until the internalization process is completed, and a higher amount of active agent could achieved the target sites.

The polymers of the invention are rapidly degraded after their internalization in the target cells as it is illustrated in the Example 16 Table 3, and Fig. 1 panel (C).

Thus, the polymers of the present invention allow the pH-dependent control of the degradation rate of the polymer in the target site, being stable until their internalization into the target cell.

The molecular weight of the polymer of the present invention in combination with the repeating units of the backbone allows the systemic distribution of the polymer and a decrease in their renal elimination. Thus, a higher proportion of non-degradable polymer is available for accumulating in the target sites.

In a preferred embodiment, the polymers of the present invention have a molecular weight of from 5,000 to 500,000 g/mol. In another preferred embodiment, the polymers of the invention have a molecular weight of from 10,000 to 200,000 g/mol. In another preferred embodiment, polymers of the present invention have a molecular weight of from 20,000 to 100,000 g/mol. In another preferred embodiment, the polymers of the invention have a molecular weight of from 35,000 to 60,000 g/mol. In a still more preferred embodiment, the polymers as mentioned above have a molecular weight about 40,000 g/mol.

Furthermore, the polymers of the invention are not toxic and biocompatible. It is shown in Example 18 which presents the comparative results of the red blood assay (RBC) of polyacetal compounds D, E, and F, using polyethylene imine (PEI) as a positive control. No RBC was observed in this assay carried out at pH 7,4 and pH 5,0 which assesses the adequate use of the polymers of the invention for intravenous administration..

In a preferred embodiment the polymers of the present embodiment are those where X is formula (II). In a particular embodiment the polymers of the invention are those where Y is formula (XI).

In another preferred embodiment, polymers of present invention are those of the following formula 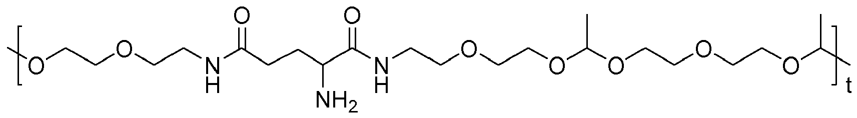 where t is an integer from 1 to 2,000

The improved pH-control of the degradation of the polymers as defined above is achieved by their molecular weight in combination with the structure of repeating units of the backbone. Polymers of the present invention are those where R₂ or R₃ is C(O)-(CH₂CH₂O)ₙ-R₅ or a saccharide, having at least a monosaccharide with a carboxylic group, selected from the group consisting of monosaccharide, disaccharide, and oligosaccharide in a percentage of from 1 to 50% by weight of the weight of the polymer. In a preferred embodiment polymers of the invention are those where R₂ or R₃ is C(O)-(CH₂CH₂O)ₙ-R₅ or the saccharide as mentioned above in a percentage of from 10 to 35%. In another more preferred embodiment polymers of the invention are those where R₂ or R₃ is C(O)-(CH₂CH₂O)ₙ-R₅ or the saccharide as mentioned above in a percentage of from 10 to 25 %.

In a particular embodiment the polymers of the invention are those where R₂ or R₃ is C(O)-(CH₂CH₂O)ₙ-R₅, and n is an integer from 1 to 300. In a preferred embodiment the polymers of the invention are those where R₂ or R₃ is C(O)-(CH₂CH₂O)ₙ-R₅, and n is an integer from 5 to 75.

In another particular embodiment the polymers of the invention are those where R₂ or R₃ is a saccharide, having at least a monosaccharide with a carboxylic group, selected from the group consisting of monosaccharide, disaccharide, and oligosaccharide. Examples of monosaccharide having a carboxylic group are selected from the group consisting of N-acetylneuraminic acid, glucuronic acid, galacturonic acid, and neuraminic acid. In a preferred embodiment the polymers of the invention are those where R₂ or R₃ is selected from the group consisting of N-acetylneuraminic acid and glucuronic acid.

The amine and hydroxy radicals of the backbone can be protected by appropriate protecting groups which allow their selective deprotection in any step of the manufacturing process. Examples of suitable amine protecting groups for the polymers of the invention include acetyl, benzyl, trifluoroacetyl, carbobenzyloxy (Cbz) and 9-fluorenylmethyloxycarbonyl (Fmoc). Preferred amine protecting groups are selected from the group consisting of acetyl, carbobenzyloxy (Cbz) and 9-fluorenylmethyloxycarbonyl (Fmoc). Examples of suitable hydroxy protecting groups for the polymer of the invention include acetyl, allyl ether, acetonide, benzoyl, benzylidenyl acetal, methoxymethyl ether (MOM), pivaloyl, tert-butyldimethylsilyl ether (TBDMS) and tert-butyldiphenylsilyl ether (TBDPS). Preferred hydroxyl protecting groups are selected from the group consisting of acetyl, acetonide and/or silyl ethers.

Another aspect of the present invention refers to a process for preparing the polymer of the present invention which comprises: a) reacting a diol selected from the group consisting of formula (XII), (XIII), (XIV), (XV), (XVI), (XVII), (XVIII), and (XIX) with a divinyl ether selected from the compound of formula (XX), and (XXI), in an anhydrous organic solvent in the presence of an acid; b) deprotecting the amine groups of the polymer-active agent conjugate obtained in step (a); and c) reacting the polymer obtained in step (b) with the compound of formula (XXII), or alternatively with a saccharide, having at least a monosaccharide with a carboxylic group, selected from the group consisting of monosaccharide, disaccharide, and oligosaccharide; where: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; R₅ is selected from the group consisting of hydrogen, and C₁-C₄ alkyl; and m, n, p, q, r, u, Z, and R₁ are as defined above.

The carboxy group of compound of formula (XXII) is bound to a R₄ which is a leaving group. Appropriate leaving groups can be an O-succinimide or an O-hydroxybenzotriazolyl ester derivative. Preferably, the leaving group is an O-succinimide derivate.

The reaction is carried out by mixing the diol and the divinyl ether at room temperature in an anhydrous organic solvent. Examples of appropriate anhydrous organic solvents are selected from the group consisting of acetonitrile (ACN), dimethyl formamide (DMF), dichloromethane (DCM), dioxane, chloroform, tetrahydrofuran, toluene and their mixtures. In a preferred embodiment, the anhydrous organic solvent is dimethyl formamide.

The process for the preparation of the polymers of the present invention is carried out in the presence of an acid. In a preferred embodiment the amount of the acid is from 0.10% to 10% by weight of the weight of the diol. In a more preferred embodiment the amount of the acid is from 0.25 to 5 % by weight of the weight of the diol. Examples of suitable acids for the preparation of the polymers as defined above are selected from the group consisting of para-toluensulfonic acid, acetic acid and hydrochloric acid (HCl). In a preferred embodiment the acid is para-toluensulfonic acid.

The reagents, active agents and starting materials are commercially available or can be prepared by any method known in the state of the art.

In addition, the polymer obtainable by the above mentioned process which comprises: a) reacting a diol selected from the group consisting of formula (XII), (XIII), (XIV), (XV), (XVI), (XVII), (XVIII), and (XIX) with a divinyl ether selected from the compound of formula (XX), and (XXI), in an anhydrous organic solvent in the presence of an acid; b) deprotecting the amine groups of the polymer-active agent conjugate obtained in step (a); and c) reacting the polymer obtained in step (b) with the compound of formula (XXII), or alternatively with a saccharide, having at least a monosaccharide with a carboxylic group, selected from the group consisting of monosaccharide, disaccharide, and oligosaccharide; where: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; R₅ is selected from the group consisting of hydrogen, and C₁-C₄ alkyl; and m, n, p, q, r, u, Z, and R₁ are as defined above, is also part of the invention.

Another aspect of the invention relates to a polymer-active agent conjugate which comprises the polymer of the present invention, and at least an active agent being covalently bound directly or by one or two linkers to the backbone as defined above. Examples of appropriate linkers for the present invention include succinimidyl, glicyl, triazole derivatives, carbamate linker, hydrazone linker, imine linker and peptide linkers suitable to be cleaved by enzymatic activity such as phenylalanyllysyl (-Phe-Lys-), glicylglicyl (-Gly-Gly-), valylcitrulyl (-Val-Cit-), and glicylphenylalanylleucylglicyl (-Gly-Phe-Leu-Gly-). Preferably, the linker is a succinimidyl.

In a preferred embodiment the polymer-active agent conjugate comprises the polymer of the present invention and the active agent is a radical attached to the amine radical of the backbone when R₂ is hydrogen or C₁-C₄ alkyl, or when Z-R₃ is NH or N(C₁-C₄ alkyl).

In another preferred embodiment the polymer-active agent conjugate comprises the polymer as defined above and the active agent is a birradical which replaces the birradical X or the birradical Y.

In another preferred embodiment the polymer-active agent conjugate comprises the polymer of the present invention, an active agent being a radical attached to the amine radical of the backbone when R₂ is hydrogen or C₁-C₄ alkyl, or when Z-R₃ is NH or N(C₁-C₄ alkyl), and an active agent being a birradical which replaces the birradical X or the birradical Y.

In a preferred embodiment the active agent is selected from the group consisting of a pharmaceutical active ingredient, a contrast agent, and a cosmetic agent.

Appropriate pharmaceutical active ingredients include, but are not limited to, anti-infective, antiseptics, anti-inflammatory agents, anti-cancer agents, antiemetics, local anesthetics, anti-acne agents, wound healing agents, and antiangiogenic agents.

Examples of pharmaceutical active ingredients suitable for being a radical attached to the amine radicals of the backbone are selected from the group consisting of: anticancer agents such as platinum complexes, floxuridine (FdUrd), gemcitabine (dFdC), deoxycytidine (dCyd), ancitabine, cyclocytidine, cytarabine, clorfarabine, trifluorothymidine, capecitabine, furtulon, tezacitabine, topotecan, 7-ethyl-10-hydroxy-camptothecin (SN-38), leuprolide, raloxifene, mitomycin C, 5-fluorouracil (5FU), methotrexate, taxanes such as paclitaxel, docetaxel, camptothecin (CPT), irinotecan (CPT-11), diethylstylbestrol (DES), curcumin, lutein, daizein, doxorubicin, and daunorubicin; antibiotic agents such as cloranfenicol, meticillin, cefazolin, and cinoxacin; anti-inflammatory agents such as prednisone, fulvestrant, and oestradiol; and carotenoids such as astaxanthin, and zeaxanthin.

Examples of pharmaceutical active ingredients suitable for being a birradical which replaces the birradical X or the birradical Y are selected from the group consisting of: anticancer agents such as platinum complexes, floxuridine (FdUrd), gemcitabine (dFdC), deoxycytidine (dCyd), ancitabine, cyclocytidine, cytarabine, clorfarabine, trifluorothymidine, capecitabine, furtulon, tezacitabine, topotecan, 7-ethyl-10-hydroxy-camptothecin (SN-38), leuprolide, diethylstylbestrol (DES), curcumin, lutein, daizein, and raloxifene; antibiotic agents such as cloranfenicol; anti-inflammatory agents such as prednisone, fulvestrant, and oestradiol; and carotenoids such as astaxanthin, and zeaxanthin.

Examples of a contrast agent suitable for being a radical attached to the amine radicals of the backbone are selected from the group consisting of triiodobenzene derivatives, ions and radioisotopes by DTPA chelating. An example of a contrast agent suitable for being a birradical which replaces the birradical X or the birradical Y is triiodobenzene derivatives.

Examples of a cosmetic agent suitable for being a radical attached to the amine radicals of the backbone are selected from the group consisting of curcumin, coenzyme Q10, D-panthenol, 1,5-pentanediol, retinol, tocoferol, tocotrienol, L-ascorbic acid, L-taurine, and nonsteroidal anti-inflammatory (NSAID). Examples of a cosmetic agent suitable for being a birradical which replaces the birradical X or the birradical Y are selected from the group consisting of curcumin, coenzyme Q10, D-panthenol, and 1,5-pentanediol.

As it is mentioned above, the degradation profile of the polymers of the present invention allows their passive accumulation at the target sites. As the polymer-active agent conjugates contain the polymer of the invention, a control in the degradation profile of the polymer-active agent conjugates is also achieved. Thus, due to the stability of the polymers of the invention in the bloodstream, and in the extracellular media, the active agent is only released from the polymer conjugate after their internalization in the target cells. It is advantageous because there is a higher amount of active agent which achieves the target sites.

As it is illustrated in Example 17 and Fig. 2, the polymers of the present invention which comprises a C(O)-(CH₂CH₂O)ₙ-R₅ or the saccharide, having a monosaccharide with a carboxylic group as defined above attached to the amine radical of the backbone (Compound E of example 9, and compound F of example 10) have a slower release of the active agent (floxuridine) than the polyacetal polymers which does not have the C(O)-(CH₂CH₂O)ₙ-R₅ chain or the saccharide (Compound D of example 8).

In particular, half of the amount of floxuridine present in Compound D, which does not form part of the invention, is released at about 60 hours. Additionally, floxuridine is practically released from Compound D at about 168 hours, while the release of floxuridine from Compounds E, and F of the present invention has been controlled. In particular, half of the amount of floxuridine present in Compound E, and F is released at about 84, and 384 hours respectively, as it is shown in Table 4.This is advantageous because the therapeutic effect of the active agent is prolonged in the time.

Another aspect of the present invention refers to a process for preparing the polymer-active agent conjugate as defined above which comprises: a) reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid; b) deprotecting the amine groups of the polymer-active agent conjugate obtained in step (a); and c) reacting the polymer-active agent conjugate obtained in step (b) with the compound selected from the group consisting of (XXII), and a saccharide having a carboxylic group; or alternatively, a') reacting the diol selected from the group consisting of formula (XII), (XIII), (XIV), (XV), (XVI), (XVII), (XVIII), and (XIX) with the divinyl ether selected from the group consisting of formula (XX), and (XXI), in an anhydrous organic solvent in the presence of an acid; b') deprotecting the amine groups of the polymer obtained in step (a'); c') reacting the polymer obtained in step (b') with the active agent; and d') reacting the polymer-active agent conjugate obtained in step (c') with the compound selected from the group consisting of (XXII), and a saccharide having a carboxylic group; or alternatively, a") reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid; b") deprotecting the amine groups of the polymer-active agent conjugate obtained in step (a"); c") reacting the polymer obtained in step (b") with the active agent; and d") reacting the polymer-active agent conjugate obtained in step (c") with the compound selected from the group consisting of (XX), and a saccharide having a carboxylic group; or alternatively, wherein: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; and m, n, p, q, r, u, Z, R₁, and R₅ are as defined above.

In addition, the polymer-active agent conjugate obtainable by the above mentioned process which comprises: a) reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid; b) deprotecting the amine groups of the polymer-active agent conjugate obtained in step (a); and c) reacting the polymer-active agent conjugate obtained in step (b) with the compound selected from the group consisting of (XXI), and a saccharide having a carboxylic group; or alternatively, a') reacting the diol selected from the group consisting of formula (XII), (XIII), (XIV), (XV), (XVI), (XVII), (XVIII), and (XIX) with the divinyl ether selected from the group consisting of formula (XX), and (XXI), in an anhydrous organic solvent in the presence of an acid; b') deprotecting the amine groups of the polymer obtained in step (a'); c') reacting the polymer obtained in step (b') with the active agent; and d') reacting the polymer-active agent conjugate obtained in step (c') with the compound selected from the group consisting of (XXII), and a saccharide having a carboxylic group; or alternatively, a") reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid; b") deprotecting the amine groups of the polymer-active agent conjugate obtained in step (a"); c") reacting the polymer obtained in step (b") with the active agent; and d") reacting the polymer-active agent conjugate obtained in step (c") with the compound selected from the group consisting of (XXII), and a saccharide having a carboxylic group; or alternatively, wherein: R₂ is selected from the group consisting of hydrogen, C₁-C₄ alkyl, C(O)-(CH₂)ᵤ-OH, and an amine protecting group; R₃ is selected from the group consisting of hydrogen, C₁-C₄ alkyl, C(O)-(CH₂)ᵤ-OH, and an amine or hydroxy protecting group; R₄ is a leaving group; and m, n, p, q, r, u, Z, R₁, and R₅ are as defined above, is also part of the invention.

In a particular embodiment the polymers and the polymer-active agent conjugates of the present invention further comprises a targeting agent being covalently bound directly or by one linker to the amine radical of the backbone, selected from the group consisting of peptide, protein, and antibody. In a preferred embodiment, the targeting agent is an antibody.

Targeting agents are agents which specifically react with an antigen or epitope of an antigen expressed on the surface of the target cell or any membrane receptor, such as a cancer or malignant cell, or against pathogens, since antibodies against pathogens are known. For example, antibodies and antibody fragments which specifically bind markers produced by or associated with infectious lesions, including viral, bacterial, fungal and parasitic infections.

Examples of targeting agents suitable for the present invention include, but are not limited to RGD peptides, and antibodies such as antibodies anti vascular endothelial growth factor (anti-VEGF), antibodies anti human epidermal growth factor receptor 2 (anti-HER₂), and antibodies anti epidermal growth factor receptor (anti-EGFR).

Polymers or polymer-active agent conjugates of the present invention can be in form of hydrogel. Thus, another aspect of the present invention is a hydrogel which comprises polymers cross-linked with a cross-linker of formula -CO-CH₂-CH₂-O-(CH₂CH₂O)ₙ-CH₂CH₂CO-, obtainable by the process which comprises cross-linking at least two polymers selected from the group consisting of: a) at least two polymers obtainable by the process defined in step (b) of third aspect; b) at least two polymers obtainable by the process defined in step (c) of third aspect, wherein the polymer obtained in step (b) is reacted with the saccharide as defined in the third aspect; c) at least two polymer-active agent conjugate obtainable by the process defined in step (b) of the fourth aspect; d) at least two polymer-active agent conjugate obtainable by the process defined in step (c) of the fourth aspect; wherein the polymer obtained in step (b) is reacted with the saccharide as defined in the third aspect; e) at least two polymer-active agent conjugate obtainable by the process defined in step (c') of the fourth aspect; f) at least two polymer-active agent conjugate obtainable by the process defined in step (d') of the fourth aspect; wherein the polymer obtained in step (c') is reacted with the saccharide as defined in the third aspect; g) at least two polymer-active agent conjugate obtainable by the process defined in step (c") of the fourth aspect; h) at least two polymer-active agent conjugate obtainable by the process defined in step (d") of the fourth aspect; wherein the polymer obtained in step (c") is reacted with the saccharide as defined in the third aspect; i) a mixture of at least two different polymers selected from (a), (b), (c), (d), (e), (f), (g), and (h); with a compound of formula (XXIII), where R₄ is as defined above; and the percentage of the cross-linker in the hydrogel is of from 0.1 to 75% by weight of the weight of the hydrogel.

Polymer hydrogels are materials composed of a solid polymer lattice capable of absorbing water. The physical or chemical bonds between the polymer chains constituting the hydrogel lattice are known as cross-links. These cross-links guarantee the structural integrity of the polymer conjugate, avoiding their complete solubilisation, and allowing the retention of the aqueous phase within the molecular mesh.

The hydrogel obtainable by the process of the present invention is a biodegradable chemical gel. A gel is defined as chemical when the network is stabilized by strong covalent bonds, formed either directly between reactive groups belonging to the polymers constituting the network, or by means of polyfunctional molecules, designated as cross-linkers, which are capable of binding several polymer chains. Hence, chemical gels are characterized by good chemical and mechanical stability as it is shown in Fig.3.

The absorbent capacity (swellability) of the lattice principally depends on the degree of hydrophilicity of the polymer and the density of cross-links. The hydrogel of the present invention allows an improved control of the release of the active agent present in the polymer- conjugate as defined above. The percentage of the cross-linker in the hydrogel of the present invention is of from 0.1 to 75% by weight of the weight of the hydrogel. When the percentage of the cross-linker is below 0.1 % the hydrogel is not formed, and if the percentage of the cross-linker is above 75% the properties of the formed hydrogel are not appropriate for the present invention.

In a preferred embodiment the percentage of the cross-linker of formula -CO-CH₂-CH₂-O-(CH₂CH₂O)ₙ-CH₂CH₂CO- is of from 5 to 60% by weight of the weight of the polymer. In another more preferred embodiment the percentage of the cross-linker as defined above is of from 10 to 50% by weight of the weight of the polymer. Preferably, the percentage of the cross-linker as defined above is of from 25 to 50% by weight of the weight of the polymer.

Active agent can be also be entrapped within the hydrogel of the present invention. Thus, the hydrogel of the present invention further comprises an active agent being not covalently bound to the backbone of the polymer, selected from the group consisting of a peptide, a protein, an antibody, and a gene construct for gene therapy. The hydrogel of the present invention also allows an improved control of the release of the active agent entrapped within the hydrogel.

In a preferred embodiment the hydrogel of the invention comprises at least a polymer-active agent conjugate cross-linked with the cross-linker of formula -CO-CH₂-CH₂-O-(CH₂CH₂O)ₙ-CH₂CH₂CO-, and another active agent being not covalently bound to the backbone of the polymer, selected from the group consisting of a peptide, a protein, an antibody, and a gene construct for gene therapy. This is advantageous because these hydrogels allow a more effective combined therapy when a multi-drug system is needed.

In a particular embodiment the hydrogel as defined above further comprises cells being not covalently bound to the hydrogel of the invention.

The polymers or polymer-active agent conjugate of the present invention can be in form of a pharmaceutical, diagnostic imaging or cosmetic composition. Thus, another aspect of the invention relates to a pharmaceutical or diagnostic imaging or cosmetic composition which comprises the polymer-active agent conjugate of the present invention, together with one or more appropriate pharmaceutically, diagnostically imaging or cosmetically acceptable excipients or carriers.

The hydrogel of the present invention can be in form of pharmaceutical, diagnostic or cosmetic composition. Thus, another aspect of the invention relates to a pharmaceutical, diagnostic or cosmetic composition which comprises the hydrogel of the present invention together with one or more appropriate pharmaceutically, diagnostically or cosmetically acceptable excipients or carriers.

Pharmaceutical, diagnostic imaging or cosmetic compositions can be in form of transdermal application or parenteral compositions for intramuscular, subcutaneous, or intravenous application. Pharmaceutical and cosmetic compositions can also be in form of topical compositions. Additionally, pharmaceutical compositions can be in form of inhalation compositions for nasally or pulmonary application.

In a preferred embodiment the compositions of the present invention are in form of parenteral compositions suitable for their injection, infusion, or implantation into the body. In another preferred embodiment the cosmetic compositions of the present invention are in form of topical or transdermal compositions.

The parenteral compositions defined above should be sterile, and pyrogen- free, and they can be in form of liquid such as solutions, emulsions, or suspensions, or in solid form packaged in either single-dose or multidose containers suitably diluted before use. Parenteral compositions can comprise appropriate excipients or carriers for parenteral administration that can be pharmaceutical or cosmetic excipients, including, but not limited to, solvents, suspending agents, buffering agents, substances to make the preparation isotonic with blood, stabilizers, or antimicrobial preservatives. The addition of excipients should be kept to a minimum. When excipients are used, they should not adversely affect the stability, bioavailability, safety, or efficacy of the polymers and/or the active agents, or cause toxicity or undue local irritation. There should not be any incompatibility between any of the components of the dosage form.

The topical compositions defined above comprise appropriate excipients or carriers for topical administration that can be pharmaceutical or cosmetic excipients, including, but not limited to, repairing cutaneous barrier function agent, a hydrating agent, an emollient, an emulsifier, a thickener, a humectant, a pH-regulating agent, an antioxidant, a preservative agent, a vehicle, or their mixtures. The excipients or carriers used have affinity for the skin, are well tolerated, stable, and are used in an amount adequate to provide the desired consistency, and ease application. Additionally, the compositions of the present invention may contain other ingredients, such as fragrances, colorants, and other components known in the state of the art for use in topical formulations. The topical compositions of the invention can be formulated in several forms that include, but are not limited to, solutions, aerosols and non-aerosol sprays, creams, powders, mousses, lotions, gels, sticks, ointments, pastes, and emulsions.

The inhalation compositions defined above can be nasally or pulmonary administered. Therefore, these compositions can be in form of nebulizer or inhaler such as nebulizer/atomizer, dry powder inhaler, nasal inhaler, or metered dose aerosol inhaler.

The above mentioned composition can be prepared according to methods well known in the state of the art. The appropriate excipients and/or carriers, and their amounts, can readily be determined by those skilled in the art according to the type of formulation being prepared.

Polymers of the present invention are intermediates for the preparation of polymer-active agent conjugates, and hydrogels of the present invention. The polymers of the invention are also useful as carriers for active-agents.

Furthermore, hydrogels which only comprises polymers of the present invention are intermediates for the preparation of hydrogels which comprises active agents being not covalently bound to the hydrogel, or can also be used as support for detection assays or dermal fillers as it is defined below.

Degradable polymer-active agent conjugates of the invention are useful in a wide variety of pharmaceutical and biomedical applications, as well as diagnostic imaging application, and in cosmetic uses.

Degradable polymer-active agents and hydrogels have enhanced properties compared to free active agents. The polymers of the invention are degradable under physiological conditions on a time-scale appropriate for the effective release of the active agent in the target sites. Furthermore, their biodistribution within the body and bloodstream is appropriate for the effective delivery of active agents for the treatment of many diseases. The polymers containing the active agents remain in the bloodstream without degradation, and do not be eliminated by the liver, but remain in circulation so as to provide the accumulation of the polymers in the target site and the release of the active agents allowing their prolonged their therapeutic effect.

Thus, an aspect of the present invention is the polymer-active agent conjugates as defined above, wherein the active agent is a pharmaceutical active ingredient for use in therapy. It also relates to a method for the prophylaxis and/or treatment of a disease which comprises administering to mammals in need of such treatment an effective amount of the polymer-active agent conjugates of the present invention, together with one or more appropriate pharmaceutically, diagnostically imaging or cosmetically acceptable excipients or carriers.

Additionally, hydrogels containing the polymer-active agent conjugates and/or active agents being not covalently bound to the hydrogel are also useful in a wide variety of pharmaceutical and biomedical applications, as well as cosmetic uses.

Another aspect of the invention is the hydrogel as defined above which comprises at least a polymer-active agent conjugate of the present invention or the active agent being not covalently bound to the hydrogel, wherein at least one of the active agent is a pharmaceutical active ingredient for use in therapy. It also relates to a method for the prophylaxis and/or treatment of a disease which comprises administering to mammals in need of such treatment an effective amount of the hydrogel which comprises at least a polymer-active agent conjugate of the present invention or the active agent being not covalently bound to the hydrogel, together with one or more appropriate pharmaceutically, diagnostically or cosmetically acceptable excipients or carriers.

In a preferred embodiment the active agent is an anti-cancer agent. Thus, the polymer-active agent conjugates for use as an anti-cancer agent, is also part of the invention. This aspect could be also formulated as the use of the polymer-active agent conjugates as defined above for the preparation of a medicament for the prophylaxis and/or treatment of a cancer. It also relates to a method for the prophylaxis and/or treatment of a cancer which comprises administering to mammals in need of such treatment an effective amount of the polymer-active agent conjugates of the present invention, together with one or more appropriate pharmaceutically excipients or carriers.

Hydrogels which comprises at least a polymer-active agent conjugate of the present invention or an active agent being not covalently bound to the hydrogel, for use as an anti-cancer agent, is also part of the invention. This aspect could be also formulated as the use of the hydrogel as defined above which comprises at least a polymer-active agent conjugate of the present invention or the active agent being not covalently bound to the hydrogel, wherein at least one of the active agent is a anti-cancer agent for the preparation of a medicament for the prophylaxis and/or treatment of a cancer. It also relates to a method for the prophylaxis and/or treatment of a cancer which comprises administering to mammals in need of such treatment an effective amount of the hydrogel as defined above which comprises at least a polymer-active agent conjugate of the present invention or the active agent being not covalently bound to the hydrogel, together with one or more appropriate pharmaceutically excipients or carriers.

In a preferred embodiment the hydrogel as defined above which comprises cells being not covalently bound to the hydrogel can be used for tissue engineering, and tissue repairing.

The polymer-active agent conjugates of the present invention can comprise a contrast agent. These contrast agents are useful as contrast imaging agents for the diagnostic imaging of a disease. The detection of these contrast agents in the body of the patient can be carried out by the well known techniques used in imaging diagnostic such as magnetic resonance imaging (MRI) and X-ray.

Thus, an aspect of the present invention is the polymer-active agent conjugates as defined above, wherein the active agent is a contrast agent, for use in diagnostic imaging. It also relates to a method for the imaging diagnostic of a disease which comprises administering to mammals in need of such diagnostic an effective amount of the polymer-active agent conjugates of the present invention, together with one or more appropriate diagnostically imaging acceptable excipients or carriers.

Another aspect of the invention is a hydrogel as defined above for use as support for detection assays. An appropriate detection assay can comprise: (a) binding the hydrogel of the invention with an agent capable of specifically binding to a target agent present in a sample to be tested; (b) putting into contact the mixture of the hydrogel and the agent of step (a) with the sample to be tested; and (c) identifying and/or quantifying the target agent.

Suitable test samples for the present invention can be serum, plasma, urine, and cephaloraquideum liquid. In a particular embodiment the identification and/or quantification of the target agent is carried out by methods well known in the art. Suitable methods for identification and/or quantification can be fluorescence, surface plasmon resonance, and colorimetry.

In a preferred embodiment the detection assays is an immunoassay which is based on the binding of an antibody and a protein. The detection assay of the invention can be used for diagnostic purposes where the present or an amount of the target agent can be correlated with a specific disease or condition.

The cosmetic composition of the invention can be used for the care of the skin. Thus, another aspect of the present invention is a use of the polymer-active agent conjugate of the present invention, wherein the active agent is a cosmetic agent as a skin care agent, wherein the skin care comprises ameliorating at least one of the following symptoms: roughness, flakiness, dehydration, tightness, chapping, and lack of elasticity.

Additionally, another aspect of the invention is the use of the hydrogel of the invention, as a skin care agent, wherein the skin care comprises ameliorating at least one of the following symptoms: roughness, flakiness, dehydration, tightness, chapping, and lack of elasticity, wherein when the hydrogel comprises an active agent, then the active agent is a cosmetic agent.

The cosmetic composition which comprises the polymer-active agent conjugate or the hydrogel as defined above is designed to apply to the body to improve its appearance or to beautify, preserve, condition, cleanse, color or protect the skin, nails or hair (cf. Academic press Dictionary of Science and Technology, 1992, pp. 531; A terminological Dictionary of the Pharmaceutical Sciences. 2007, pp.190). Therefore, the above cosmetic compositions are adjectivally used for a non-medical application. In a preferred embodiment, the cosmetic composition as defined above can be used for topical or systemic or parenteral application to the skin as anti-wrinkle agent, skin anti-ageing agent, photoprotector agent, and dermal filler agent.

Throughout the description and claims the word "comprises" and variations of the word, are not intended to exclude other technical features, additives, components or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

The following abbreviations are used in the below examples:
- ACN: acetonitrile
- Asp: aspartic acid
- DCM: dichloromethane
- DES: diethylestylbestrol
- DIEA: diisopropylethylamine
- DMF: dimethylformamide
- DMSO: dimethylsulfoxide
- DOX: doxorubicin
- equiv.: equivalent
- FdUrd: floxuridine
- Fmoc: 9-fluorenylmethylcarbonyl
- Glu: glutamic acid
- h: hours
- HOAt: 1-hydroxy-7-azabenzotriazole
- HOBt: 1-hydroxybenzotriazole
- HPLC: high performance liquid chromatography
- HPLC-MS(ESI): high performance liquid chromatography - mass spectrometry (electrospray)
- MeO-PEG-NHS: methyloxide polyethyleneglycol O-succinimidoester
- MHz: megaherz
- Mw: molecular weight
- Mn: number average molecular weight
- MWCO: molecular weight cut-off
- NANA: N-acetyl-neuraminic acid
- NHS: N-hydroxysuccinimide
- NHS-PEG-NHS: di-O-succinimidoester polyethyleneglycol
- NMR: nuclear magnetic resonance
- PEG: polyethylene glycol
- pTSA: para-toluene sulfonic acid
- SEC-MALS-RI: size exclusion chromatography - multi angle light scattering - refractive index
- SEM: scanning electron microscopy
- Ser: serine
- t: time
- tBu: tert-butyl
- THF: tetrahydrofurane
- TPA: ter-polyacetal
- TFA: trifluoroacetic acid
- Tyr: tyrosine
- WSC: water soluble carbodiimide
- % w/w: percentage in weight

### (A) Preparation of monomers:

### Example 1: Preparation of Diol 1 derived from glutamic acid (Fmoc-Glu-Dm (₂CO₂C) Dol)

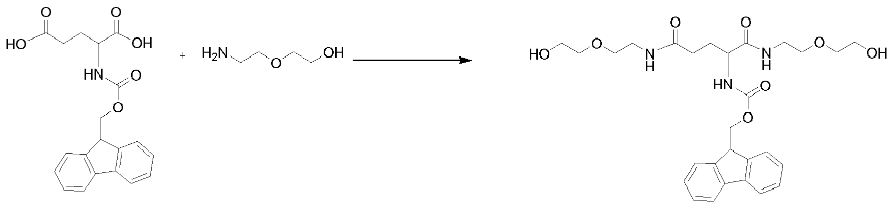

A solution of Fmoc-Glu-OH (3 g, 8.12 mmol), WSC.HCl (4.8 g, 25.07 mmol) and HOAt (3.3 g, 24.24 mmol) in DMF (24 mL) in a 50 mL round-bottom flask was stirred at room temperature for 15 min. Then, 2-(2-aminoethoxy)-ethanol (1.63 mL, 15.58 mmol) was added. The mixture was gently stirred for 15 min at r.t. The final solution was diluted in DCM (70 mL) and transferred to a separatory funnel, and washed with water (4x100 mL). The organic layer was dried over Na₂SO₄ filtered and the solvent removed by rotaevaporation to afford the product (a white wax, 70%). Mw 544.2 g/mol. ¹H-NMR (400MHz, dmso-d6): 1.75 (1H, m), 1.85 (1H, m), 2.12 (2H, m), 3.20 (4H, m), 3.40 (8H, m), 3.48 (4H, m), 3.94 (1H, m), 4.22 (3H, m), 4.56 (2H, t), 7.33 (2H, t), 7.42 (2H, t), 7.48 (1H, d), 7.73 (2H, m), 7.83 (1H, t), 7.92 (3H, m).

### Example 2: Preparation of diol 2 derived from serine (Fmoc-Ser-Am(5C)Ol

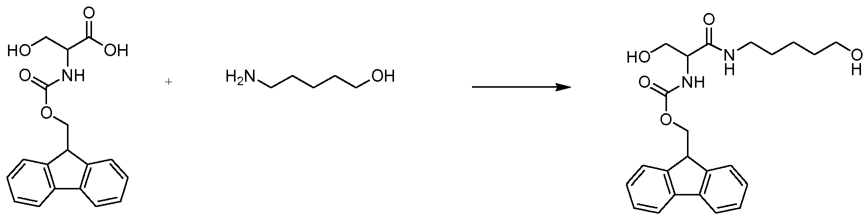

A solution of Fmoc-Ser-OH (1 g, 3.17 mmol), WSC.HCl (1.17 g, 6.11 mmol) and HOBt (0,82 g, 6.07 mmol) in DMF (7 mL) in a 25 mL round-bottom flask was stired at room temperature for 3 min. Then, 5-amino-1-pentanol (0.314 g, 3.04 mmol) was added. The mixture was gently stirred for 15 min at room temperature. The final solution was added dropwise on water (100 ml) and the white product precipitates. Once the water was decanted, the wet solid was lyophilized (Quantitative yield). Mw 412.5 g/mol. ¹H-NMR (400MHz, dmso-d6): 1.26 (2H, m), 1.39 (4H, m), 1.61 (2H, m), 3.05 (2H, m), 3.35 (2H, m), 3.55 (2H, m), 4.00 (1 H, m), 4.22 (1 H, m), 4.26 (2H, m), 4.32 (1 H, t), 4.83 (1H, t), 7.22 (1H, d), 7.33 (2H, t), 7.42 (2H, t), 7.75 (2H, d), 7.79 (1H, m), 7.89 (2H, d).

### Example 3: Preparation of diol 3 derived from tyrosine ([Fmoc-Tyr]₂-PEG)

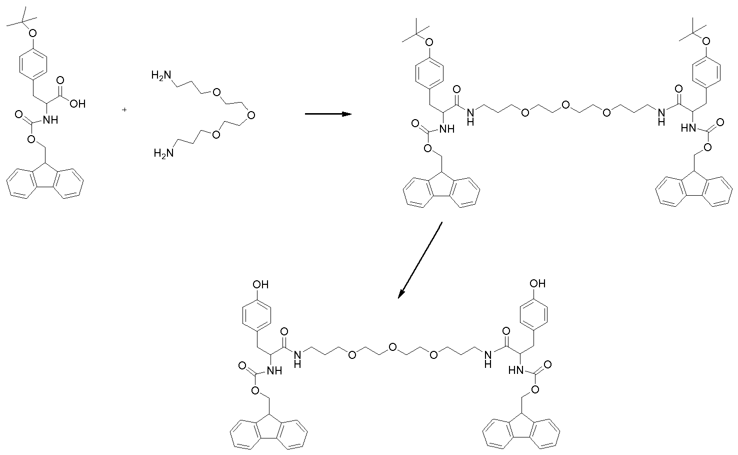

A solution of Fmoc-Tyr(tBu)-OH (0.5 g, 1.09 mmol), WSC.HCl (0.4 g, 2.12 mmol) and HOBt (0,29 g, 2.19 mmol) in DMF (3 mL) in a 10 mL round-bottom flask was stirred at room temperature for 10 min. Then, 4,7,10-trioxa-1,13- tridecanediamine (0.12 ml, 0,547 mmol) was added dropwise during 15 min. The reaction mixture was stirred for 15 min at room temperature. The solvent was removed by rotatory evaporation. The crude was solved in 35 ml DCM and washed with 4x50 ml of water. The organic layer was dried over Na₂SO₄, filtered and the solvent removed by rotaevaporation to afford the product (a white solid, 97%). Deprotection of tert-butyl protecting groups was carried out by solving the product obtained before in 10 ml TFA:DCM 1:1 and stirring the solution for 30 min. at room temperature. Then, the solvent was evaporated under N2 and the resulting crude was suspended in a low volume of 0.2 M HCl:ACN 1:1 and lyophilized (96 %). Mw 991.1 g/mol. ¹H-NMR (400MHz, dmso-d6): 1.56 (4H, m), 2.66 (1H, dd), 2.80 (1H, dd), 3.31 (2H, t), 3.43 (16H, m), 4.07 (2H, m), 4.12 (4H, d), 4.18 (2H, s), 4.21 (1H, m), 6.61 (4H, d), 7.03 (4H, d), 7.29 (4H, d), 7.39 (4H, t), 7.65 (4H, t), 7.87 (4H, d), 9.07 (2H, s).

### Example 4: Preparation of diol 4 derived from serine ([Fmoc-Ser]₂-PEG)

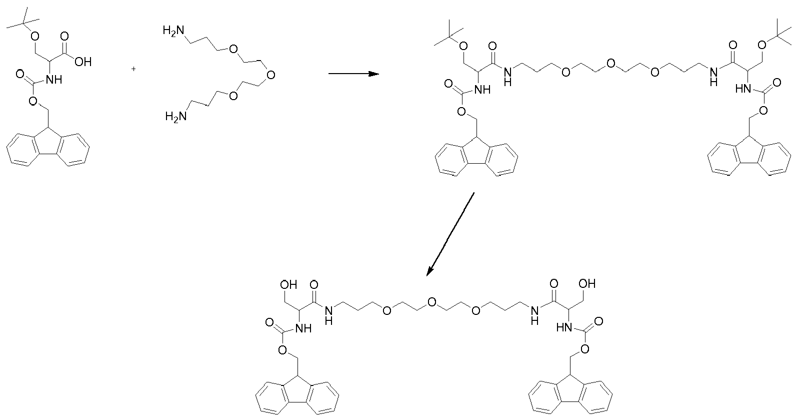

Diol 4 was carried out following the process described in example 3 using the following starting materials: Fmoc-Ser(tBu)-OH (0.6 g, 1.56 mmol), WSC.HCl (0.6 g, 3.13 mmol), HOBt (0.36 g, 2.66 mmol) and 4,7,10-trioxa-1,13-tridecanediamine (0.17 ml, 0.78 mmol). Diol 4 yields 70%. Mw: 838.9 g/mol, ¹H-NMR (400MHz, dmso-d6): 1.61 (4H, m), 3.10 (2H, m), 3.44 (12H, t), 3.55 (4H, m), 3.99 (2H, m), 4.21 (2H, t), 4.26 (4H, d), 5.10 (2H, m), 7.31 (4H, t), 7.41 (4H, t), 7.73 (4H, d), 7.88 (4H, d).

### Example 5: Preparation of divynil ether 1 derived from aspartic acid Fmoc-Asp-Dm(3C)Dv

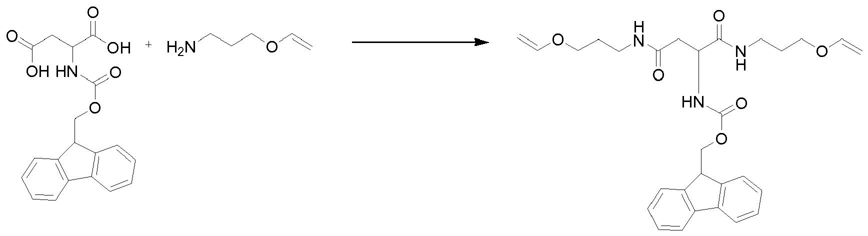

A solution of Fmoc-Asp-OH (0.5 g, 1.41 mmol), WSC.HCl (1.07 g, 5.62 mmol) and HOBt (0,76 g, 5.62 mmol) in DMF (3 mL) in a 10 mL round-bottom flask was stirred at room temperature for 3 min. Then, 3-ethenyloxy propan-1-amine (0.28 g, 2.81 mmol) was added. The reaction mixture was stirred for 15 min at room tempearture. The DMF was removed by rotatory evaporation. The crude obtained was solved in 35 ml DCM (purified by alumina) and washed with 3x50 ml of 50 mM (NH₄)₂CO₃. The organic layer was dried over Na₂SO₄, filtered and the solvent removed by rotaevaporation to afford the product (a pale yellow solid, 90%). Mw 521.6 g/mol. ¹H-NMR (400MHz, dmso-d6): 1.70 (4H, m), 2.38 (1H, m), 2.47 (1H, m), 3.10 (4H, m), 3.65 (4H, m), 3.93 (1H, dd), 4.14 (2H, dd), 4.24 (4H, m), 6.46 (2H, dd), 7.31 (2H, t), 7.41 (2H, t), 7.70 (2H, d), 7.89 (2H, d).

### Example 6: Preparation of divynil ether 2 derived from glutamic acid Fmoc-Glu-Dm(3C)Dv

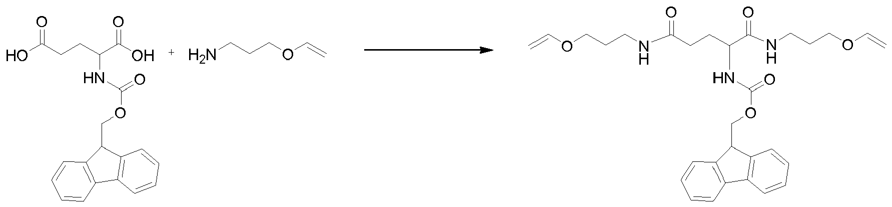

Divinyl ether 2 was carried out following the process described in example 5 using the following starting materials: Fmoc-Glu-OH (0.7g, 1.89mmol), WSC.HCl (1.45g, 7.57mmol), HOBt (1g, 7.40 mmol), 3-ethenyloxy propan-1-amine (0.38 g, 3.78 mmol). Divinyl ether 2 yields 88%. Mw: 535.6 g/mol, ¹H-NMR (400MHz, dmso-d6): 1.70 (5H, m), 1.85 (1H, m), 2.09 (2H, m), 3.14 (4H, m), 3.64 (4H, t), 3.89 (1H, m), 3.93 (2H, dd), 4.13 (2H, dd), 4.21 (3H, m), 6.46 (2H, dd), 7.31 (2H, t), 7.39 (2H, t), 7.71 (2H, d), 7.87 (2H, d).

### (B) Preparation of Polymers-active agents conjugates

### Example 7: Preparation of Compound C: Polymer-DES-PEG conjugate. ([TPA(DES)]gPEG_{5kDa})

### A) Preparation of Compound B: Polymer-DES conjugate (TPA(DES)

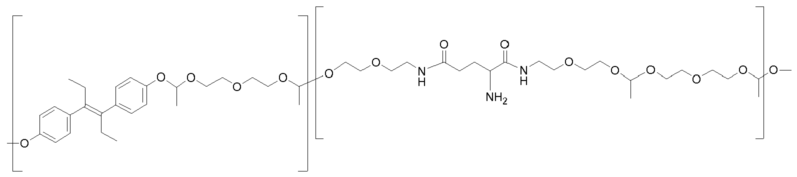

A solution of pTSA (dried overnight under vacuum at 35 °C, 13 mg, 0.068 mmol), DES (83.5 mg, 0.311 mmol) and the diol 1 of example 1 (0.501 g, 0.922 mmol) in 0.5 ml DMF (anhydride) was stirred for 10 min. at room temperature, in the presence of molecular sieve (4 amstrong), in a 10 mL round-bottom flask (dried overnight at 110 °C), which was sealed with a septum and purged with Argon. Then, diethylene glycol divinyl ether (200 µL, 1.22 mmol) was added and the mixture was stirred slowly in the dark. Up to an additional equivalent of diethylene glycol divinyl ether was added during the polymerization process. A viscous solution was obtained in 3 h. The reaction was quenched with Et₃N (170 µL) and stirred for 10 min at room temperature. The viscous solution was precipitated in Et₂O (50 mL) and washed again 2x50 mL with additional Et₂O until a white solid was obtained (600 mg). Once the polymer was dried under vacuum, it was solved in 20% piperidine in ACN (15 mL) and shaked at room temperature for 1 h. The resulting solution was diluted with (NH₄)₂CO₃ 50 mM. A white solid precipitated and it was removed by centrifugation and filtration of the supernatant. The final clear solution was lyophilized. The white solid obtained was solved in 20% ACN in (NH₄)₂CO₃ 50 mM (300 mL) and filtered through a controlled porous membrane (MWCO 10 kDa) in an Amicon 8400 (Millipore®) system by application of Argon pressure (2-3 bar). The solution was concentrated to 75 mL and purification/filtration process was carried out three times more. The final concentrate was lyophilized. The molecular weight was Mw = 43 kDa and Mw/Mn = 1.76 as determined by SEC-MALS RI (dn/dc = 0.135). ¹H-NMR (400MHz, dmso-d6): 1.17 (3H, d, acetal CH₃) and 4.68 (1H, q, acetal CH) for the acetal formed by diol 1 of example 1 and diethylene glycol divinyl ether and 1.40 (3H, d, acetal CH₃) and 5.48 (1H, q, acetal CH) for the acetal formed by DES and diethylene glycol divinyl ether. The diol 1:DES ratio quantified by ¹H-NMR was 3:1. (14.4 % w/w DES).

### B) Preparation of Compound C: Polymer-DES-PEG conjugate [TPA(DES)]gPEG_{5kDa}

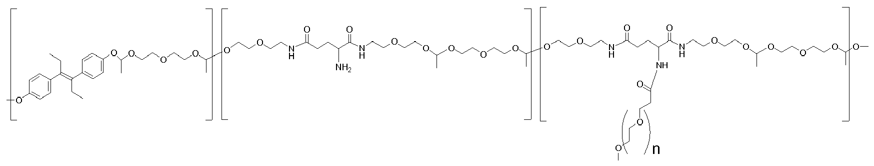

The polymer obtained in section A (43kDa, 75 mg) were solved in 1 ml DMF. Then, MeO-PEG-NHS (5000 Da, 3.71 mg, 2.9 mmol) as solid was added. The final mixture was stirred overnight in the dark at room temperature. The final reaction mixture was diluted in 15 ml 50mM (NH₄)₂CO₃ buffered to pH 8-9 and the polymer was purified by ultracentrifugation through a controlled porous membrane (MWCO 10 kDa, 5x15ml, 3000g) in an Amicon Ultra (Millipore®) system. The final solution was lyophilized. The molecular weight was Mw = 50.7 kDa and Mw/Mn = 1.47 as determined by SEC-MALS RI (dn/dc = 0.135). ¹H-NMR (400MHz, dmso-d6): 1.17 (3H, d, acetal CH₃) and 4.68 (1H, q, acetal CH) for the acetal formed by diol 1 of example 1 and diethylene glycol divinyl ether and 1.40 (3H, d, acetal CH₃) and 5.48 (1H, q, acetal CH) for the acetal formed by DES and diethylene glycol divinyl ether. DES was quantified by acidic hydrolysis (in 0.2 M HCl) and analysis of degraded mixture by HPLC (using resorcinol as internal standard): DES (11.9 % w/w, 139.5 µmol/g).

### Example 8: Preparation of compound D: Polymer-floxuridine conjugate (TPA(FdUrd))

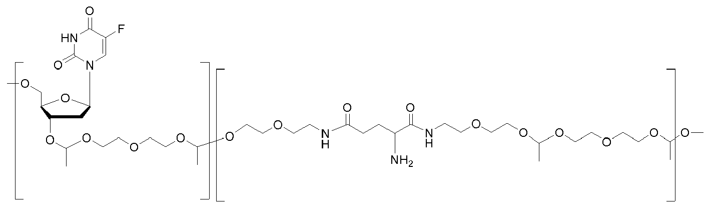

A solution of pTSA (dried overnight under vacuum at 35 °C, 23.4 mg, 0.123 mmol), FdUrd (70 mg, 0.284 mmol) and the diol 1 of example 1 (1 g, 1.843 mmol) in 1.2 ml DMF (anhydride) was stirred for 10 min at room temperature, in the presence of molecular sieve (4 amstrong), in a 10 mL round-bottom flask (dried overnight at 110 °C), which was sealed with a septum and purged with Argon. Then, diethylene glycol divinyl ether (350 µL, 2.14 mmol) was added and the mixture was stirred slowly in the dark. Up to an additional equivalent of diethylene glycol divinyl ether were added during the polymerization process. A viscous solution was obtained in 3 h. The reaction was quenched with Et₃N (320 µL) and stirred for 10 min. at room temperature. The viscous solution was precipitated in Et₂O (50 mL) and washed again 4x50 mL with additional Et₂O until a white solid was obtained (1.6 g). Once the polymer was dried under vacuum, it was solved in 20% piperidine in ACN (15 mL) and shaked atroom temperature for 1 h. The resulting solution was diluted with (NH₄)₂CO₃ 50 mM. A white solid precipitated and it was removed by centrifugation and filtration of the supernatant. The final clear solution was lyophilized. The white solid obtained was solved in 20% ACN in (NH₄)₂CO₃ 50 mM (300 mL) and filtered through a controlled porous membrane (MWCO 10 kDa) in an Amicon 8400 (Millipore®) system by application of Argon pressure (2-3 bar). The solution was concentrated to 75 mL and purification/filtration process was carried out three times more. The final concentrate was lyophilized. The molecular weight was Mw = 41.2 kDa and Mw/Mn = 3.1 as determined by SEC-MALS RI (dn/dc = 0.135). ¹H-NMR (400MHz, dmso-d6): 1.17 (3H, d, acetal CH₃) and 4.68 (1H, q, acetal CH) for the acetal formed by diol 1 of example 1 and diethylene glycol divinyl ether and 1.21 (3H, d, acetal CH₃) and 4.76 (1H, q, acetal CH) for the acetal formed by FdUrd and diethylene glycol divinyl ether. The diol 1:FdUrd ratio was analyzed by acidic hydrolysis (in 0.2 M HCl) and analysis of degraded mixture by HPLC (using resorcinol as internal standard): diol 1:FdUrd 10:1 (4,63% w/w FdUrd, 188,2 µmol/g).

### Example 9: Preparation of Compound E: Polymer-floxuridine-PEG conjugate [TPA(FdUrd)]gPEG_{5KDa}

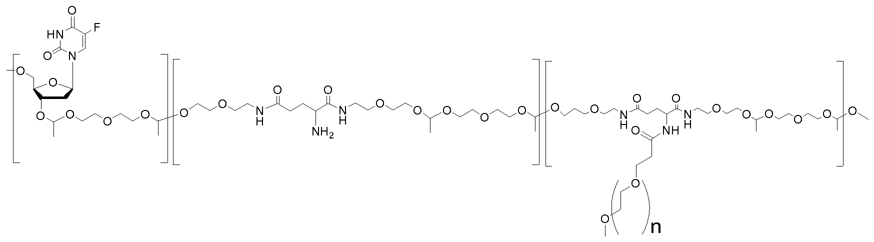

The polymer obtained in example 8 (41.2 kDa, 51.2 mg, 1.24 µmol) was solved in 0.5 ml DMF and it was added over a solution of MeO-PEG-NHS (5 kDa, 23.8 mg, 4,76 µmol) in 0.5 ml 50 mM NaHCO₃ (pH 8). The mixture was stirred overnight in the dark at room temperature. The final reaction mixture was diluted in 15 ml 50mM (NH₄)₂CO₃ buffered at pH 8-9 and the polymer was purified by ultracentrifugation through a controlled porous membrane (MWCO 10 kDa, 5x15ml, 3000g) in an Amicon Ultra (Millipore®) system. The final solution was lyophilized. The molecular weight was Mw = 60.9 kDa and Mw/Mn = 2.8 as determined by SEC-MALS RI (dn/dc = 0.135). The FdUrd was quantified by acidic hydrolysis (in 0.2 M HCl) and analysis of degraded mixture by HPLC (using resorcinol as internal standard): FdUrd 3.51 % w/w, 142.8 µmol/g).

### Example 10: Preparation of Compound F: Polymer-floxuridine-N-acetylneuraminic acid conjugate. [TPA(FdUrd)]-NANA.

N-acetyl-neuraminic acid (NANA) (37 mg, 0.119 mmol) was activated with WSC.HCl (68.4 mg, 0.357 mmol) and HOAt (48.6 mg, 0.357 mmol) in 1 ml DMF for 20 min. This solution was added to a solution of the polymer of example 8 (41.2 kDa, 98.3 mg, 2.386 µmol) in 1 ml DMF. The mixture was stirred overnight in the dark at room temperature. The final reaction mixture was diluted in 15 ml 50mM (NH₄)₂CO₃ buffered at pH 8-9 and the polymer was purified by ultracentrifugation through a controlled porous membrane (MWCO 3 kDa, 5x15ml, 3000g) in an Amicon Ultra (Millipore®) system. The final solution was lyophilized. The molecular weight was Mw = 65.7 kDa and Mw/Mn = 3.2 as determined by SEC-MALS RI (dn/dc = 0.135). The FdUrd was quantified by acidic hydrolysis (in 0.2 M HCl) and analysis of degraded mixture by HPLC (using resorcinol as internal standard): FdUrd 3.56% w/w, 144.8 µmol/g).

### Example 11: Preparation of polymer-floxuridine-doxorubicin conjugate TPA(FdUrd)-DOX

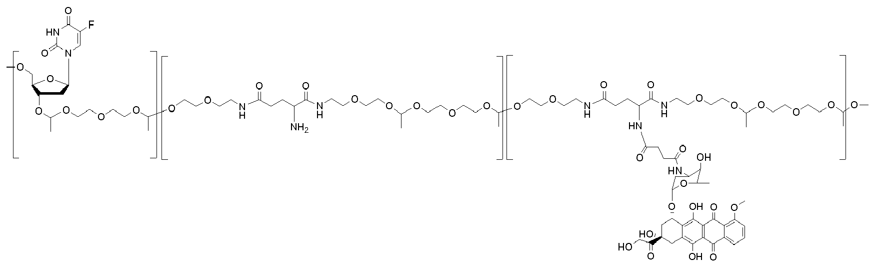

Hydrochloride doxorubicin (6.23 mg, 0,011 mmol) was solved in 0.3 mL DMF and 1.90 µl DIEA were added. The mixture was shaked at room temperature for 5 min. Then, 0.1 ml DMF solution of succinic anhydride (1.07 mg, 0,011 mmol) was added. The mixture was shaked at room temperature for 2 h in the dark to afford the succinylated doxorubicin (Mw 644 g/mol, cheked by HPLC-MS(ESI)). WSC.HCl (6.1 mg, 0.032 mmol) and HOAt (4.4 mg, 0.032 mmol) were added as solids on the succinylated Doxorubicin solution in DMF, and the reaction mixture was shaked at room temperature for 20 min. Then, the mixture was added on a solution of the polymer of example 8 in 0.5 ml DMF. The resulting mixture was diluted with 0.4 ml DMF and it was stirred overnight in the dark at room temperature. The mixture was diluted in 15 ml 50mM (NH₄)₂CO₃ buffered at pH 8-9 and the polymer was purified by ultracentrifugation through a controlled porous membrane (MWCO 10 kDa, 5x15ml, 3000g) in an Amicon Ultra (Millipore®) system. The final solution was lyophilized. The molecular weight (only soluble portion) was Mw = 31.5 kDa and Mw/Mn = 1.29 as determined by SEC-MALS RI (dn/dc = 0.135). FdUrd content was analyzed by acidic hydrolysis (in 0.2 M HCl) and analysis of degraded mixture by HPLC (using resorcinol as internal standard): 4.41 % w/w FdUrd). Doxorubicin content was analyzed by UV(300nm): 9.06% w/w

### Example 12: Preparation of polymer-floxuridine-doxorubicin-PEG conjugate TPA(FdUrd)-DOX-PEG2K

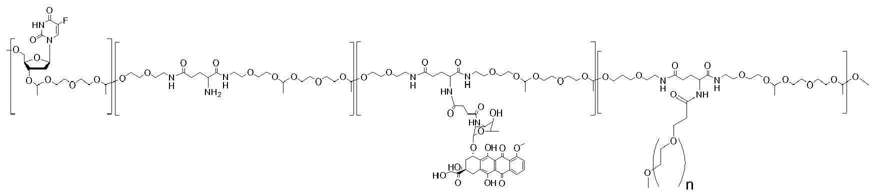

Hydrochloride doxorubicin-(12 mg, 0,021 mmol) was solved in 0.4 mL DMF and 3.80 µl DIEA were added. The mixture was shaked at room temperature for 5 min. Then, 0.1 ml DMF solution of succinic anhydride (2.07 mg, 0.021 mmol) was added. The mixture was shaked at room temperature for 2 h in the dark to afford the succinylated Doxorubicin (Mw 644 g/mol, cheked by HPLC-MS(ESI)). WSC.HCl (12.1 mg, 0.063 mmol) and HOAt (8.9 mg, 0.065 mmol) were added as solids on the succinylated Doxorubicin solution in DMF, and the reaction mixture was shaked at room temperature for 20 min. Then, the mixture was added on a solution of the polymer of example 8 in 1 ml DMF. The total mixture was diluted with a 1 ml DMF and it was stirred overnight in the dark at room temperature. MeO-PEG-NHS 2000 Da (65.1 mg, 12.36 mmol) were added as a solution in 0.5 ml 50 mM NaHCO₃ (pH 8). The resultant mixture was stirred overnight in the dark at room temperature. HPLC-MS(ESI) control showed the incorporation of PEG to the polymer (observing delta-Mw of 44 Da in the polymer peak). The mixture was diluted in 15 ml 50mM (NH₄)₂CO₃ buffered at pH 8-9 and the polymer was purified by ultracentrifugation through a controlled porous membrane (MWCO 10 kDa, 5x15ml, 3000g) in an Amicon Ultra (Millipore®) system. The final solution was lyophilized. The molecular weight (only soluble portion) was Mw = 39.7 kDa and Mw/Mn = 1.59 as determined by SEC-MALS RI (dn/dc = 0.135). FdUrd content was analyzed by acidic hydrolysis (in 0.2 M HCl) and analysis of degraded mixture by HPLC (using resorcinol as internal standard): 2.92% w/w FdUrd.). Doxorubicin content was analyzed by UV(300nm): 5.54% w/w

### Example 13: Preparation of polymer-floxuridine-doxorubicin-N-acetyl neuraminic acid conjugate. TPA(FdUrd)-DOX-NANA

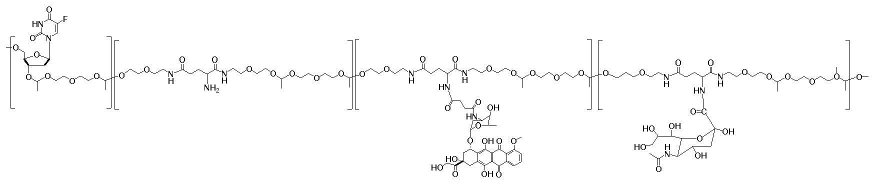

Hydrochloride doxorubicin (6 mg, 0.010 mmol) was solved in 0.15 mL DMF and 1.90 µl DIEA were added. The mixture was shaked at room temperature for 5 min. Then, 0.1 ml DMF solution of succinic anhydride (1.03 mg, 0.010 mmol) was added. The mixture was shaked at room temperature for 2 h in the dark to afford the succinylated Doxorubicin (Mw 644 g/mol,cheked by HPLC-MS(ESI)). WSC.HCl (6 mg, 0.031 mmol) and HOAt (4.5 mg, 0.033 mmol) were added as solids on the succinylated Doxorubicin solution in DMF, and the reaction mixture was shaked at room temperature for 20 min. Then, the mixture was added on a solution of the polymer of example 8 in 1 ml DMF. The total mixture was stirred overnight in the dark at room temperature. N-acetyl-neuraminic acid (NANA) (21 mg, 0.065 mmol) was activated with WSC.HCl (38.8 mg, 0.203 mmol) and HOAt (27.5 mg, 0.202 mmol) in 0.5 ml DMF for 20 min. Then, this solution was added to a solution of the TPA(FdUrd)-DOX. The mixture was stirred for 6 h in the dark at room temperature. The final mixture was diluted in 15 ml 50mM (NH₄)₂CO₃ buffered at pH 8-9 and the polymer was purified by ultracentrifugation through a controlled porous membrane (MWCO 10 kDa, 5x15ml, 3000g) in an Amicon Ultra (Millipore®) system. The final solution was lyophilized. The molecular weight was Mw = 47.2 kDa and Mw/Mn = 3.3 as determined by SEC-MALS RI (dn/dc = 0.135). FdUrd content was analyzed by acidic hydrolysis (in 0.2 M HCl) and analysis of degraded mixture by HPLC (using resorcinol as internal standard): 2.29% w/w FdUrd.). Doxorubicin content was analyzed by UV(300nm): 4.83% w/w.

### (C) Preparation of hydrogel

### Example 14: Preparation of a hydrogel derived from Compound D of example 8 crosslinked with PEG

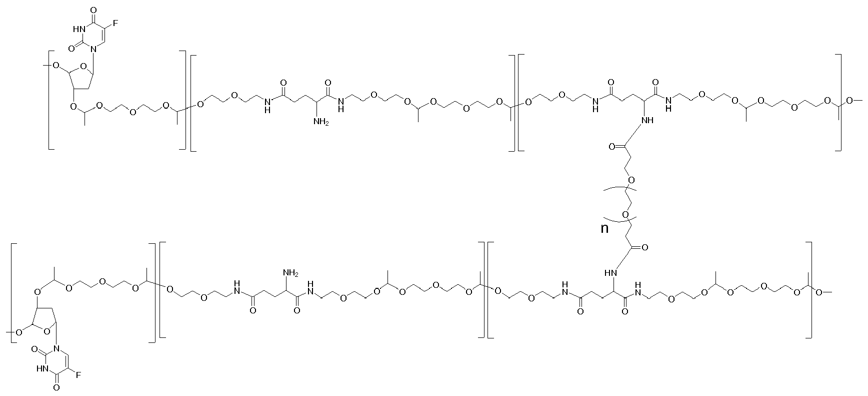

The polymer obtained in example 8 (50 mg) was solved in 0.1 ml 50 mM NaHCO₃ (pH 8). Then, NHS-PEG-NHS (10000 Da, 50 mg) was added as solid. The resulting mixture was gently shaked until a homogeneous viscous solution is obtained. 0.3 ml of additional buffer was added when viscosity was too high. The hydrogel was formed rapidly. The resulting jelly was left at 4°C overnight. The material was washed with 10 ml 50mM (NH₄)₂CO₃ buffer (pH 8-9) (cycles of swelling and centrifugation). The final swollen hydrogel was lyophilized. Scaning electron micrsocopy (SEM) was used to corroborate the sponge-like structure of hydrogels obtained.

### (D) Preparation of reference standard polymer

### Example 15. Compound A: Polyacetal of PEG (PA)

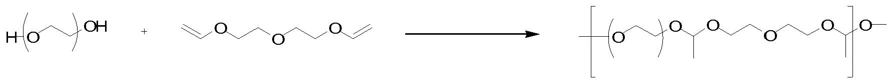

A solution of pTSA (dried overnight under vacuum at 45 °C, 9.9 mg, 0.052 mmol) and PEG (2000 Da, 1 g, 0.5 mmol) in 1.4 ml THF (anhydride) was stirred for 10 min at room temperature, in the presence of molecular sieve (4 amstrong), in a 10 mL round-bottom flask (dried overnight at 110 °C), which was sealed with a septum and purged with Argon. Then, diethylene glycol divinyl ether (123 µL, 0.753 mmol) was added and the mixture was stirred slowly in the dark. Up to an additional equivalent of diethylene glycol divinyl ether were added during the polymerization process. A viscous solution was obtained in 3 h. The reaction was quenched with Et₃N (130 µL) and stirred for 10 min at room temperature. The viscous solution was precipitated in Et₂O (50 mL) and washed again 2x50 mL with Et₂O until a white solid was obtained. The resultant solid was solved in 20% ACN in (NH₄)₂CO₃ 50 mM (300 mL) and filtered through a controlled porous membrane (MWCO 10 kDa) in an Amicon 8400 (Millipore®) system by application of Argon pressure (2-3 bar). The solution was concentrated to 75 mL and purification/filtration process was carried out three times more. The final concentrate was lyophilized. The molecular weight was Mw = 62.5 kDa and Mw/Mn = 1.76 as determined by SEC-MALS RI (dn/dc = 0.135). ¹H-NMR (400MHz, dmso-d6): 1.18 (3H, d, acetal CH₃) and 4.70 (1 H, q, acetal CH).

### Example 16: Degradation study of polymers of the present invention

The degradation rate of the following polymers was tested:
- Compound A (PA): polyacetal polymer without no active agent in the backbone, and without C(O)-(CH₂CH₂O)ₙ-R₅ or saccharide chains attached to the amine radicals of the backbone (Example 15);
- Compound B (TPA(DES)): polymer in which DES is inserted in the backbone without C(O)-(CH₂CH₂O)ₙ-R₅ or saccharide chains attached to the amine radicals of the backbone (Example 7 section A); and
- Compound C ([TPA(DES)]gPEG_{5kDa}): polymer in which DES is inserted in the backbone and C(O)-(CH₂CH₂O)ₙ-R₅ chains attached to the amine radicals of the backbone (Example 7 section B).

Compound A does not have free amine radicals in the backbone, and Compound B contains the free amine radical, but does not have the C(O)-(CH₂CH₂O)ₙ-R₅ or saccharide chains attached to the above-mentioned amine radicals. Thus, both Compounds A and B do not form part of the invention.

The stability of the above-mentioned compounds is tested at different pH:
- pH of the bloodstream (pH 7.4),
- pH of the extracellular media (pH 6.5), and
- pH of the intracellular media (endosomes) (pH 5.5).

For determining the degradation rate of the compounds of the study at the above-mentioned pH, the quantification of the loss of the molecular weight of the polymer in due time (Mwₜ) is compared with the molecular weight of the polymer at time zero (Mwₒ) was determined. Thus, the ratio (Mwₜ)/(Mwₒ) shows the percentage of molecular lost at each pH according to the time.

The method for determining the degradation profile of Compounds A, B, and C comprises dissolving 15 mg/ml of the tested compound in aqueous buffers at pH 7.4 (in PBS), pH 6.5 (in 0.1 M phosphate buffer), or pH 5.5 (in 0.1 M phosphate buffer). Then, the resultant solutions at 37 °C were incubated. Aliquots of 200 µl were taken at different times during 1 month.

These aliquots were analyzed by a SEC-MALS-RI: Wyatt DAWN EOS multi angle light scattering detector operating at 632.8 nm using a SEC column consisting of Ultrahydrogel 500 (Waters); and refractive index detector (Waters 2414, delay volume 250 µl), and the evaluation of the collected data was accomplished by ASTRA software v.4.90.07.

### Conditions of the elution:

- Mode: Isocratic
- Eluent: (NH₄)₂CO₃ (50 mM) and ACN (20%)
- Flow rate: 0.7 ml/min
- Eluents were filtered through 0.22 µm, degassed and gassed with dried helium
- Analysis temperature: 30°C
- Calibrated with toluene (HPLC grade) and normalized with BSA and dextran standards
- Specific refractive index increment: dn/dc = 0.185 ml/g

The results of the degradation rate of the tested compounds are summarized in the following tables:

**Table 1. Loss of molecular weight of compounds A, B, and C at pH 7.4**

| | **Compound A** | | **Compound B** | | **Compound C** | |
|---|---|---|---|---|---|---|
| t (days) | (Mwₜ)/(Mwₒ) | SD | (Mwₜ)/(Mwₒ) | SD | (Mwₜ)/(Mwₒ) | SD |
| 0 | 100 | 0 | 100 | 0 | 100 | 0 |
| 1 | 102 | 7.47 | 94.33 | 7.04 | 99.35 | 7.19 |
| 2 | 95.15 | 2.66 | 91.93 | 6.68 | 101.32 | 2.05 |
| 3 | - | - | 89.47 | 3.87 | 96.3 | 2.1 |
| 4 | 83.98 | 5.16 | - | - | - | - |
| 7 | 73.43 | 4.35 | 87.35 | 7.04 | 95.59 | 5.09 |
| 10 | - | - | 83.15 | 1.95 | 86.64 | 4.49 |
| 11 | 65.45 | 1.69 | - | - | - | - |
| 14 | 56.71 | 4.18 | 78.77 | 1.27 | 81.6 | 2.21 |
| 21 | **43.03** | 2.29 | **69.25** | 5.98 | **75.39** | 2.39 |
| 30 | **31.46** | 1.87 | **60.43** | 3.38 | **65.23** | 2.69 |

| | | | | | | |
|---|---|---|---|---|---|---|
| *SD: standard deviation | | | | | | |

The previous results in Table 1 and in Fig. 1 panel (A) show that Compounds B, and C which comprises amine radical in the backbone are stables in the bloodstream conditions because about 70% of the molecular initial weight of the polymer is not degraded. In particular, Compound B has a 69.25% of the initial molecular weight, and Compound C has a 75.39% of the initial molecular weight after 21 days. While Compound A which does not contain free amine groups in the backbone has only about a 40% of the initial molecular weight of the polymer at the same time.

Thus, the loss of the molecular weight of the Compound C of the present invention is a 35% less than in Compound A, and a 6% less than in Compound B. It means that the polymers of the present invention are more stable in the bloodstream than the polymers of the state of the art. This is advantageous because the non-degradation of the polymers of the invention involves that a higher amount of the active agent is retained in them.

**Table 2. Loss of molecular weight of compounds A, B, and C at pH 6.5**

| | **Compound A** | | **Compound B** | | **Compound C** | |
|---|---|---|---|---|---|---|
| t (days) | (Mwₜ)/(Mwₒ) | SD | (Mwₜ)/(Mwₒ) | SD | (Mwₜ)/(Mwₒ) | SD |
| 0 | 100 | 0 | 100 | 0 | 100 | 0 |
| 1 | 63.18 | 4.05 | 66.73 | 3.36 | 82.59 | 2.36 |
| 2 | 49.55 | 1.35 | 55.48 | 6.23 | 76.33 | 9.61 |
| 3 | - | - | 48.52 | 4.73 | 65.17 | 1.82 |
| 4 | 34.14 | 4.51 | - | - | - | - |
| 7 | 20.4 | 2.45 | 42.58 | 8.51 | 59.37 | 0.62 |
| 10 | - | - | 31.02 | 3.13 | 55.88 | 1.83 |
| 11 | 16.87 | 2.13 | - | - | - | - |
| 14 | 16.51 | 0.84 | 23.68 | 5.06 | 46.01 | 3.57 |
| 21 | **9.75** | 0.57 | **12.41** | 1.33 | **36.63** | 0.51 |
| 30 | **6.82** | 1.49 | **9.62** | 2.69 | **29.67** | 4.37 |

| | | | | | | |
|---|---|---|---|---|---|---|
| *SD: standard deviation | | | | | | |

The previous results in Table 2 and in Fig. 1 panel (B) show that Compound C of the present invention which comprises C(O)-(CH₂CH₂O)ₙ-R₅ chains attached to the amine radicals of the backbone is more stable in the extracellular media than Compound B which does not contain C(O)-(CH₂CH₂O)ₙ-R₅ chains, and much more stable than Compound A. In particular, Compound C maintains a 36.63% of the initial molecular weight after 21 days, whereas the initial molecular weight of Compound A, and Compound B is reduced to a 9.75%, and a 12.41 % respectively.

Thus, the loss of the molecular weight of the Compound C of the present invention is about 27% less than in Compound A, and about 24% less than in Compound B. It means that polymers of the present invention are more stable in the extracellular media than the polymers of the state of the art. This is advantageous because the polymers of the present invention are more stables during the internalization process of the polymer, allowing the internalization of a higher amount of the active agent retained in the polymer conjugate.

**Table 3. Loss of molecular weight of compounds A, B, and C at pH 5.5**

| | **Compound A** | | **Compound B** | | **Compound C** | |
|---|---|---|---|---|---|---|
| t (days) | (Mwₜ)/(Mwₒ) | SD | (Mwₜ)/(Mwₒ) | SD | (Mwₜ)/(Mwₒ) | SD |
| 0 | 100 | 0 | 100 | 0 | 100 | 0 |
| 1 | 19.8 | 3.78 | 20.89 | 2.19 | 67.33 | 2.8 |
| 2 | 9.9 | 1.62 | 17.27 | 5.35 | 29.19 | 1.88 |
| 3 | - | - | 14.09 | 1.44 | 25.57 | 6.47 |
| 4 | 9.24 | 4.67 | - | - | - | - |
| 7 | 3.84 | 2.32 | 11.93 | 2.98 | 20.74 | 1.52 |
| 10 | - | - | 6.4 | 1.29 | 13.7 | 1.02 |
| 11 | 2.73 | 0.69 | - | - | - | - |
| 14 | **2.27** | 0.55 | **6.14** | 0.96 | **12.14** | 2.25 |
| 21 | 2.47 | 0.35 | 1.82 | 0.22 | 6.71 | 1.12 |
| 30 | 2.32 | 0.35 | 4.54 | 0.77 | 6.76 | 1.41 |

| | | | | | | |
|---|---|---|---|---|---|---|
| *SD: standard deviation | | | | | | |

The previous results in Table 3 and in Fig.1 panel (C) show that Compound C of the present invention is rapidly degraded after their internalization in the target site. In particular, Compound C has a 12.14% of the initial molecular weight after 14 days, whereas the initial molecular weight in Compound A, and in Compound B is reduced to a 2.27%, and a 6.14% respectively. It means that the rapid degradation of the polymer facilitate the release of the active agent covalently bound to the backbone.

This degradation test makes evident the importance of the existence of the amine radicals and the C(O)-(CH₂CH₂O)ₙ-R₅ or saccharide chains presents in the polymers of the invention to avoid the degradation of the polymer during the systemic circulation in the bloodstream, accumulation in the target sites and the internalization process, whereas the polymer is rapidly degraded inside the target cells.

### Example 17: Study of the release of floxuridine from polymers of the present invention

The release of floxuridine from the following polymers was tested:
- Compound D (TPA(FdUrd)): polymer in which floxuridine is inserted in the backbone of the polymer (Example 8);
- Compound E ([TPA(FdUrd)]gPEG_{5kDa}): polymer in which floxuridine is inserted in the backbone and C(O)-(CH₂CH₂O)ₙ-R₅ chains are attached to the amine radicals of the backbone (Example 9); and
- Compound F (TPA(FdUrd)-NANA): polymer in which floxuridine is inserted in the backbone and N-acetylneuraminic acid chains are attached to the amine radicals of the backbone (Example 10).

Compound A does not contain C(O)-(CH₂CH₂O)ₙ-R₅ or saccharide chains attached to the free amine radicals in the backbone. Thus, compound A does not form part of the invention.

The release of floxuridine from the above-mentioned compounds is tested at different pH:
- pH of the bloodstream (pH 7.4) shown as dotted line;
- pH of the extracellular media (pH 6.5) shown as dashed line; and
- pH of the intracellular media (endosomes) (pH 5.5) shown as solid line.

The method for determining the release rate of floxuridine from the above-mentioned compounds at the specified pH comprises dissolving 10 mg/ml of the tested compound in aqueous buffers at pH 7.4 (in PBS), pH 6.5 (in 0.1 M phosphate buffer), or pH 5.5 (in 0.1 M phosphate buffer). Then, the resultant solutions at 37 °C were incubated. Aliquots of 20 µl were taken at different times during 1 week, and the aliquots were quenched with 20 µl of 50 mM (NH₄)₂CO₃ at pH 8-9.

Aliquots were analyzed by HPLC: Alliance 2695 (Waters) and photodiode array detector 2998 (Waters) operating at 280 nm, using a XBridge column (Waters) C18, 3.5 µm, 4.6x100 mm.

### Conditions of the elution:

- Mode: gradient,
- Eluents: Eluent A: water 0.45% TFA, and Eluent B: ACN 0.36% TFA
- Gradient: from 0 to 50 % of eluent B
- Time of the gradient: 8 min.
- Temperature: room temperature
- Flow rate: 1 ml/min
- Internal standard: Resorcinol

The results of the release rate of floxuridine from the tested compounds are summarized in Table 4.

**Table 4. Release rate of floxuridine**

| | **Compound D** | | | **Compound E** | | | **Compound F** | | |
|---|---|---|---|---|---|---|---|---|---|
| | pH 5.5 | | pH 6.5 | pH 5.5 | | pH 6.5 | pH 5.5 | | pH 6.5 |
| t (hour) | (%) | SD | (%) | (%) | SD | (%) | (%) | SD | (%) |
| 0 | - | - | - | - | - | - | - | - | - |
| 1 | - | - | - | - | - | - | - | - | - |
| 8 | 0.17 | 0.0 1 | 0.08 | 0.22 | 0 | - | 0.1 | - | - |
| 24 | 2.91 | 0.0 9 | - | 1.72 | 0.02 | - | 0.4 | 0.1 | - |
| 48 | 21.95 | 0.5 4 | - | 11.18 | 0.59 | - | 0.75 | 0.02 | 0.12 |
| **72** | **64.4** | 2.3 | - | **35.6** | 2.31 | - | **1.37** | 0.05 | 0.15 |
| 96 | 79.54 | 0.8 4 | 0.03 | 63.89 | 0.76 | - | 2.14 | 0.01 | 0.17 |
| **168** | **100.96** | 1.7 5 | 0.04 | **96.16** | 1.32 | - | **6.8** | 0.3 | 0.36 |
| 216 | - | - | - | - | - | - | 12.8 | 0.1 | 0.54 |
| 264 | - | - | - | - | - | - | 18.9 | 0.42 | 0.71 |
| 336 | - | - | - | - | - | - | 29.6 | 0.56 | 1 |
| 360 | - | - | - | - | - | - | 40.0 7 | 0.69 | 1.36 |
| 384 | - | - | - | - | - | - | 48.2 8 | 0.34 | 2 |

| | | | | | | | | | |
|---|---|---|---|---|---|---|---|---|---|
| *SD standard deviation | | | | | | | | | |

There is no release of floxuridine in any of the tested compounds at pH 7.4. It means that the active agent is not released from the polymer when the polymer-active agent conjugate is in the bloodstream. And, the release of floxuridine at pH 6.5 was practically negligible in all tested compounds for a period of time lower than 168 hours.

The previous results in Table 4 and in Fig. 2 panel (A) show that half of the amount of floxuridine present in Compound D is released at about 60 hours, and it is practically released at about 168 hours at intracellular conditions (pH 5.5). The release of floxuridine from compounds E, and F of the present invention has been controlled. In particular, half of the amount of floxuridine present in compound E, and F is released at about 84, and 384 hours respectively.

This release test makes evident that polymers of the invention can modulate the release of the active agent. It is advantageous because an appropriate release profile for each active agent can be achieved.

### Example 18. Red Blood cell lysis assay

The red blood assay (RBC) is carried out using the same compounds D, E, and F as in the degradation study in Example 17. Triton X-1 00 (1 % solution) was used as a reference control to measure 100% of hemolysis, polyethyleneimine (PEI) and dextran were used as positive and negative controls respectively.

The blood was extracted from Wistar rats and placed in heparinized tubes. Then, the blood was diluted with PBS pH 7.4 up to 10 ml, and centrifuged (3000 rpm) for 10 minutes at 4°C for three times. The supernatant was removed after each centrifugation. The sediment thus obtained was used to prepare 3 RBC solutions at 2% w/v in PBS at pH 7.4 and 5.0.

To determine the hemolytic activity of tested compounds or their degradation products, four concentrations of the above-mentioned compounds were prepared: 0.5, 1, 1.5 and 2 mg/ml which were placed in 96 well micro-titer plates. Then, RBC solution was added before incubation.

After 24 h, samples were centrifuged (3000 rpm) for 10 min. at room temperature and the supernatant of each well was transferred in another plate to measure the absorbance of released heamoglobin at 570 nm using a micro-titer plate reader. The degree of lysis was expressed as a percentage of the Triton X-1 00 value.

No absorbance was observed in any of the tested compounds D, E, and F. Thus, none of the tested compounds promote the lysis of red blood cells.

### Example 19. SEM micrographs

1-2 mg of the hydrogel material was hydrated in 0.1 M phosphate buffer pH 7.4 at room temperature for 3h. The sample was then placed in a glass container and frozen in liquid nitrogen for 10 minutes. Then, the material thus obtained was lyophilized and dried. Finally, the dried material was placed on a standard microscope support to be scanned in a Nova NANOSEM230 (Field Emission) at low vacuum mode (for non-conducting materials): HV:5kV.

### REFERENCES CITED IN THE APPLICATION

- European patent number EP1315777
- Academic press Dictionary of Science and Technology, 1992, pp. 531
- A terminological Dictionary of the Pharmaceutical Sciences. 2007, pp.190.

## Claims

1. A polymer having a molecular weight of from 1,000 to 1,000,000 g/mol which comprises a backbone of repeating structural units, being these repeating structural units equals or differents of formula (I), 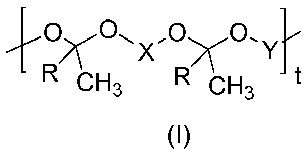 wherein:
R is a radical independently selected from the group consisting of hydrogen, and C₁-C₄ alkyl ;
X is a birradical selected from the group consisting of formula (II), (III), (IV), (V), (VI), (VII), (VIII), and (IX); 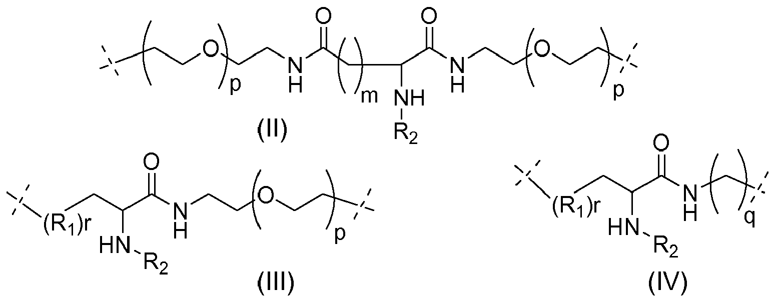 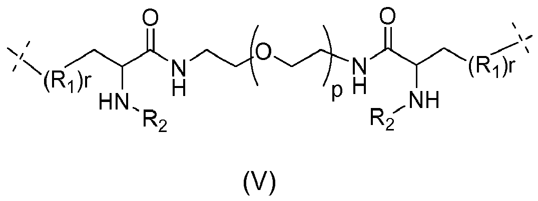 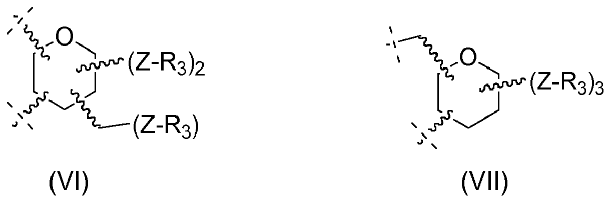 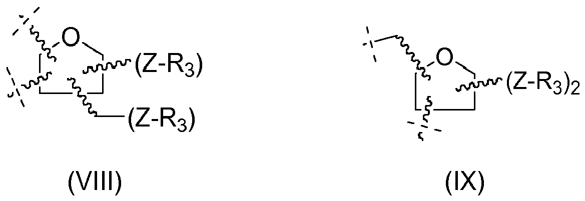
Y is a birradical selected from the group consisting of formula (X), and (XI); 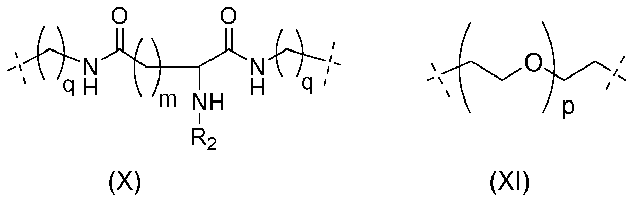
Z is a heteroatom selected from the group consisting of N and O;
R₁ is a phenyl group;
R₂ is selected from the group consisting of hydrogen, C₁-C₄ alkyl, C(O)-(CH₂)ᵤ-OH, an amine protecting group, C(O)-(CH₂CH₂O)ₙ-R₅, and a saccharide, having at least a monosaccharide with a carboxylic group, selected from the group consisting of monosaccharide, disaccharide, and oligosaccharide;
R₃ is selected from the group consisting of hydrogen, C₁-C₄ alkyl, C(O)-(CH₂)ᵤ-OH, an amine or hydroxy protecting group, C(O)-(CH₂CH₂O)ₙ-R₅, and a saccharide, having at least a monosaccharide with a carboxylic group, selected from the group consisting of monosaccharide, disaccharide, and oligosaccharide, with the proviso that when Z is O then R₃ is a hydroxyl protecting group, hydrogen or C₁-C₄ alkyl;
R₅ is selected from the group consisting of hydrogen, and C₁-C₄ alkyl;
m is an integer from 1 to 2;
n is an integer from 1 to 300;
p is an integer from 1 to 20;
q is an integer from 1 to 20;
r is an integer from 0 to 1;
t is an integer from 1 to 2,000;
u is an integer from 1 to 20;
the wavy line indicates any of the two possible configurations of the radicals attached to the carbocycle of formula (VI), (VII), (VIII), and (IX);
the intermittent line indicates the position through which the birradicals X and
Y bind to the oxygen atoms of the repeating structural units of the backbone,
with the proviso that R₂ or R₃ is C(O)-(CH₂CH₂O)ₙ-R₅ or the saccharide in a percentage of from 1 to 50% by weight of the weight of the polymer.

2. The polymer according to claim 1, wherein the molecular weight of the polymer is of from 20,000 to 100,000 g/mol.

3. The polymer according to any of claims 1-2, wherein X is formula (II).

4. The polymer according to claim 3, wherein the compound of formula (I) is: 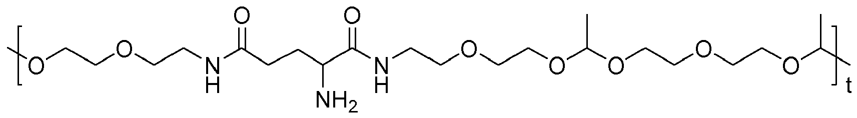 wherein t is as defined in claim 1.

5. A polymer-active agent conjugate which comprises the polymer as defined in any of the claims 1-4 and at least an active agent being covalently bound directly or by one or two linkers to the backbone,
wherein:
a) the active agent is a radical attached to the amine radical of the backbone when R₂ is hydrogen or C₁-C₄ alkyl, or when Z-R₃ is NH or N(C₁-C₄ alkyl), or alternatively,
b) the active agent is a birradical which replaces the birradical X or the birradical Y; or alternatively,
c) a mixture of a) and b).

6. The polymer-active conjugate according to claim 5, wherein the active agent is selected from a pharmaceutical active ingredient, a contrast agent, and a cosmetic agent.

7. A process for preparing a polymer as defined in any of the claims 1-4, which comprises:
a) reacting a diol selected from the group consisting of formula (XII), (XIII), (XIV), (XV), (XVI), (XVII), (XVIII), and (XIX) 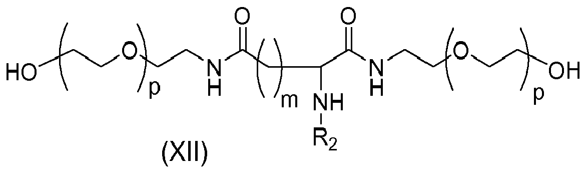 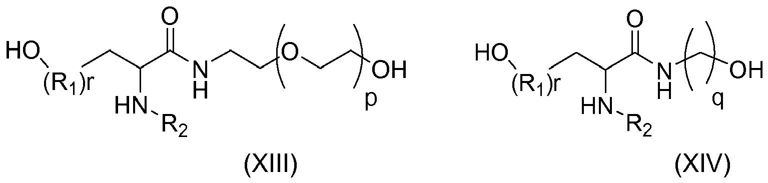 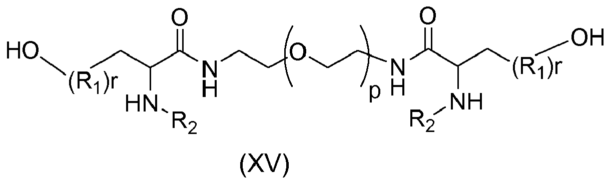 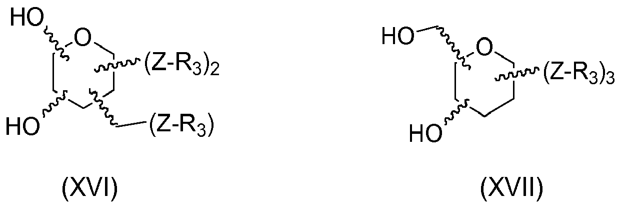 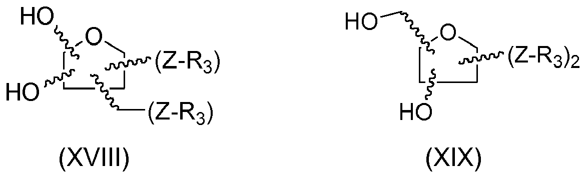 with a divinyl ether selected from the compound of formula (XX), and (XXI), 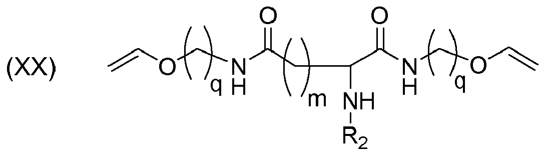 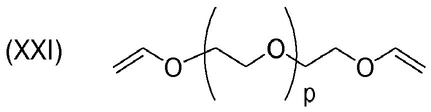 in an anhydrous organic solvent in the presence of an acid;
b) deprotecting the amine groups of the polymer-active agent conjugate obtained in step (a); and
c) reacting the polymer obtained in step (b) with the compound of formula (XXII), 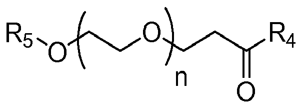 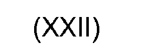 or alternatively with a saccharide, having at least a monosaccharide with a carboxylic group, selected from the group consisting of monosaccharide, disaccharide, and oligosaccharide;
wherein:
R₂ is selected from the group consisting of hydrogen, C₁-C₄ alkyl, C(O)-(CH₂)ᵤ-OH, and an amine protecting group;
R₃ is selected from the group consisting of hydrogen, C₁-C₄ alkyl, C(O)-(CH₂)ᵤ-OH, and an amine or hydroxy protecting group;
R₄ is a leaving group;
R₅ is selected from the group consisting of hydrogen, and C₁-C₄ alkyl; and
m, n, p, q, r, u, Z, and R₁ are as defined in claim 1.

8. A process for preparing a polymer-active agent conjugate as defined in any of the claims 5-6 which comprises:
a) reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid;
b) deprotecting the amine groups of the polymer-active agent conjugate obtained in step (a); and
c) reacting the polymer-active agent conjugate obtained in step (b) with the compound selected from the group consisting of (XXII), and a saccharide having a carboxylic group; or alternatively,
a') reacting the diol selected from the group consisting of formula (XII), (XIII), (XIV), (XV), (XVI), (XVII), XVIII), and (XIX) with the divinyl ether selected from the group consisting of formula (XX), and (XXI), in an anhydrous organic solvent in the presence of an acid;
b') deprotecting the amine groups of the polymer obtained in step (a');
c') reacting the polymer obtained in step (b') with the active agent; and
d') reacting the polymer-active agent conjugate obtained in step (c') with the compound selected from the group consisting of (XXII), and a saccharide having a carboxylic group; or alternatively,
a") reacting the diol selected from the group consisting of formula (XII), (XIII), (XIV), (XV), (XVI), (XVII), (XVIII), and (XIX); with the divinyl ether selected from the group consisting of formula (XX), and (XXI); and the active agent in an anhydrous organic solvent in the presence of an acid;
b") deprotecting the amine groups of the polymer-active agent conjugate obtained in step (a");
c") reacting the polymer obtained in step (b") with the active agent; and
d") reacting the polymer-active agent conjugate obtained in step (c") with the compound selected from the group consisting of (XXII), and a saccharide having a carboxylic group; or alternatively,
wherein:
R₂ is selected from the group consisting of hydrogen, C₁-C₄ alkyl, C(O)-(CH₂)ᵤ-OH, and an amine protecting group;
R₃ is selected from the group consisting of hydrogen, C₁-C₄ alkyl, C(O)-(CH₂)ᵤ-OH, and an amine or hydroxy protecting group;
R₄ is a leaving group; and
m, n, p, q, r, u, Z, R₁, and R₅ are as defined in claim 1.

9. A pharmaceutical or diagnostic imaging or cosmetic composition which comprises the polymer-active agent conjugate as defined in any of the claims 5-6, together with one or more appropriate pharmaceutically, diagnostically imaging or cosmetically acceptable excipients or carriers.

10. A polymer-active agent conjugate as defined in any of the claims 5-6, wherein the active agent is a pharmaceutical active ingredient for use in therapy.

11. The polymer-active agent conjugate according to claim 10, for use as an anti-cancer agent.

12. A polymer-active agent conjugate as defined in any of the claims 5-6, wherein the active agent is a contrast agent for use as diagnostic imaging agent.

13. Use of the polymer-active agent conjugate as defined in any of the claims 5-6, wherein the active agent is a cosmetic agent as a skin care agent, wherein the skin care comprises ameliorating at least one of the following symptoms: roughness, flakiness, dehydration, tightness, chapping, and lack of elasticity.

14. A hydrogel comprising polymers cross-linked with a cross-linker of formula -CO-CH₂-CH₂-O-(CH₂CH₂O)ₙ-CH₂CH₂CO-, obtainable by the process which comprises cross-linking at least two polymers selected from the group consisting of:
a) at least two polymers obtainable by the process defined in step (b) of claim 7;
b) at least two polymers obtainable by the process defined in step (c) of claim 7, wherein the polymer obtained in step (b) is reacted with the saccharide as defined in claim 7;
c) at least two polymer-active agent conjugate obtainable by the process defined in step (b) of claim 8;
d) at least two polymer-active agent conjugate obtainable by the process defined in step (c) of claim 8; wherein the polymer obtained in step (b) is reacted with the saccharide as defined in claim 7;
e) at least two polymer-active agent conjugate obtainable by the process defined in step (c') of claim 8;
f) at least two polymer-active agent conjugate obtainable by the process defined in step (d') of claim 8; wherein the polymer obtained in step (c') is reacted with the saccharide as defined in claim 7;
g) at least two polymer-active agent conjugate obtainable by the process defined in step (c") of claim 8;
h) at least two polymer-active agent conjugate obtainable by the process defined in step (d") of claim 8; wherein the polymer obtained in step (c") is reacted with the saccharide as defined in claim 7;
i) a mixture of at least two different polymers selected from (a), (b), (c), (d), (e), (f), (g), and (h);
with a compound of formula (XXIII), 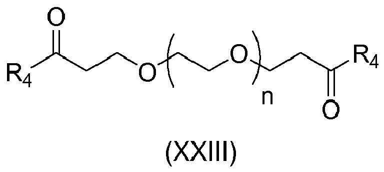 wherein
R₄ is as defined in claim 1; and the percentage of the cross-linker in the hydrogel is of from 0.1 to 75 % by weight of the weight of the hydrogel.

15. The hydrogel according to claim 14, further comprising an active agent being not covalently bound to the backbone of the polymer, selected from the group consisting of a peptide, a protein, an antibody, and a gene construct for gene therapy.

16. A pharmaceutical, diagnostic or cosmetic composition which comprises the hydrogel as defined in any of the claims 14-15, together with one or more appropriate pharmaceutically, diagnostically or cosmetically acceptable excipients or carriers.

17. A hydrogel which comprises at least a polymer-active agent conjugate as defined in any of the claims 5-6 or the active agent as defined in claim 15, wherein at least one active agent is a pharmaceutical active ingredient for use in therapy.

18. The hydrogel according to claim 17, for use as an anti-cancer agent.

19. The hydrogel according to any of the claims 14-15, for use as a support for detection assays.

20. Use of the hydrogel as defined in any of the claims 14-15, as a skin care agent, wherein the skin care comprises ameliorating at least one of the following symptoms: roughness, flakiness, dehydration, tightness, chapping, and lack of elasticity, wherein when the hydrogel comprises an active agent, then the active agent is a cosmetic agent.
